# EUROPEAN PATENT SPECIFICATION

(11) **EP 4 103 158 B1**
(45) Date of publication and mention of the grant of the patent: **04.09.2024**
(21) Application number: 21705458.4
(22) Date of filing: 10.02.2021
(51) Int. Cl.: A61K 9/20, A61K 9/16, A61K 31/403, A61K 31/404

(54) **COMPOSITION COMPRISING RAMIPRIL AND INDAPAMIDE**
ZUSAMMENSETZUNG MIT RAMIPRIL UND INDAPAMID
COMPOSITION COMPRENANT DU RAMIPRIL ET DE L'INDAPAMIDE

(30) Priority: 10.02.2020 EP 20156309
(43) Date of publication of application: 21.12.2022
(73) Proprietor: Adamed Pharma S.A., 05-152 Czosnow (PL)
(72) Inventor: KIELPINSKI, Mateusz, 87-100 Torun (PL); RZASA, Joanna, 35-116 Rzeszow (PL); KUBIAK, Bartlomiej, 05-101 Nowy Dwor Mazowiecki (PL); DABROWSKA, Justyna, 47-430 Ruda Kozielska (PL)
(74) Representative: Matusiewicz, Katarzyna Joanna
(86) International application number: PCT/EP2021/053251
(87) International publication number: WO 2021/160700

(56) References cited:
- WO-A1-2008/124611
- WO-A1-2020/109319
- WO-A2-2005/074884
- US-A1- 2010 062 062

## Description

### Field of the present invention

The present invention relates to a pharmaceutical composition comprising ramipril and indapamide for oral administration, which can be used for treating of hypertension.

### Technological background

Ramipril, an angiotensin converting enzyme (ACE) inhibitor, is intended for the treatment of hypertension. Ramipril can be used as a monotherapy or can be co-administered with other cardiovascular drug substances as the individual products given concurrently in the separate unit dosage forms or at the same dose level as the fixed-dose combination. Such approved combined treatment is the combination of immediate release Ramipril and sustained release of Indapamide for oral administration.

For best compliance fixed dose combination of immediate release Ramipril and sustained release of Indapamide is preferred over administration of separate dosage forms of Ramipril and Indapamid. Such a fixed dose may be used in therapy only when bioequivalence in comparison to both referent medicines is confirmed. Meaning that concurrently requirements of chemical stability fulfilled and dissolution profiles resemble that of the respective referent drugs. Otherwise the fixed dose combination is not suitable to be used as a medicine.

Document WO2008124611 discloses fixed dosage forms of Ramipril and Indapamid. However, in fact two separate forms of ramipril and indapamide are placed in a hard capsule. As a result only large capsule sizes are able to contain quantities of the medicines and as a result difficulties in swallowing are very likely to occur. This inconvenience is not to be omitted in case of hypertension therapy which last over prolonged period of time. Additionally, in such capsule, the two particles types are in contact, which results in degradation of both active ingredients. Besides, the manufacturing process is time consuming and expensive.

In WO2020109319 a bilayer tablet of Ramipril and Indapamide is disclosed. The drug form overcomes the inconveniences connected with a large capsule. Chemical stability of both active ingredients has been presented for the variants of tablets where Ramipril layer is stabilized with sodium hydrogen carbonate. However, no experimental dissolution data is provided. Another drawback is that the total mass of the ramipril layer is not diversified between particular dosages, which results in the same wt.% of excipients for all ramipril strengths and finally results in unnecessary amounts of fillers used and finally unnecessarily big tablets for lower ramipril strengths, causing inconveniences of patients due to the size of the tablet but also due to unnecessary intake of excipient substances. Economical losses resulting from this are not to be diminished.

The instability of Ramipril is generally known in the art. It is ascribed to a combination of such factors as heat, moisture, oxidation and in the case of tablets, the compression processes used in manufacture. Efforts to stabilize ramipril have focused on protective coatings, and the use of select binders, and/or additives. However, the proposed solutions rarely proof to be working. Especially when ramipril is combined with another, chemically not compatible active substances. As it is in the case of indapamide.

Well known are also the general problems of bilayer tablets resulting from influences of the respective layers components and their matrix-formation behaviour on the dissolution profile of the active ingredient of the other layer. Up now these problems have not been addressed for bilayer tablets of ramipril and indapamide.

In EP20156309 of the present Applicant granulates of Ramipril stabilized with PVA are disclosed. The granulates remain stable when further be compressed to tablets.

Still however exist a need of fixed dosage form comprising ramipril and indapamide which could be readily replace administration of separate dosage forms of ramipril and indapamide.

### Summary of the invention

It is an object of the present invention to provide a pharmaceutical composition of Ramipril and Indapamide that reduces or overcomes the above-mentioned problems and may readily be used as a single dose alternative in treatments requiring separate ramipril and indapamide administration.

The object is solved by pharmaceutical composition comprising Ramipril and Indapamide according to the appended claims.

In particular, the present invention relates to the following aspects:
(1) According to an aspect of the present invention, the pharmaceutical composition for oral administration is a single dosage form provided in form of a bilayer tablet, the tablet comprising:
   a) an immediate release layer comprising Ramipril and polyvinyl alcohol in a wt.% ratio of 1.1 to 4.0 and one or more pharmaceutically acceptable excipients; and
   b) a sustained-release layer, comprising Indapamid, a sustained-release agent selected from hydroxypropylmethylcellulose (HPMC) or hydroxyethylcellulose (HEC) grades with a viscosity in a range of 2 500 - 5 500 mPas, measured according to EU Pharmacopoeia 7.0 method 2.2.8 in 2wt.% water solution, and one or more pharmaceutically acceptable excipients.
(2) The pharmaceutical composition according to (1), wherein ramipril is contained as free acid, or a pharmaceutically acceptable salt of the free acid; and indapamide is contained as free amide, or a pharmaceutically acceptable salt of the free amide, preferably ramipril is contained as the free acid and indapamide is contained as the free amide.
(3) The pharmaceutical composition according to (1) or (2), wherein in the immediate release layer a) the wt.% ratio of ramipril to polyvinyl alcohol is in a range of 1.1 to 4.0, preferably 1.15 to 3.9, more preferably 1.7 to 2.85, such as 1.75 or 2.8.
(4) The pharmaceutical composition according to (3), wherein the tablet is uncoated or coated. Preferably, the tablet is coated.
(5) The pharmaceutical composition according to (4), wherein the immediate release layer a) comprises 10, 5 or 2.5 mg of ramipril calculated on a ramipril free acid, and the sustained-release layer b) comprises 1.5 mg of indapamid, calculated on indapamide free amide; and, preferably, the immediate release layer a) comprises 10 or 5 mg of ramipril, and the sustained-release layer b) comprises 1.5 mg of indapamid.
(6) The pharmaceutical composition according to (5), wherein the immediate release layer a) has a ramipril content of 4.5 to 5.5 wt.%, preferably 4.5 to 5.0 wt.%, more preferably 4.5 to 4.9 wt.%, such as 4.9 wt.%, based on the total weight of the immediate release layer a); furthermore, the sustained release layer b) has a indapamide content of 0.5 to 1.0 wt.%, preferably 0.6 to 0.9 wt.%, more preferably 0.6 to 0.8 wt.%, such as 0.75 wt.%, based on the total weight of the sustained-release layer b).
(7) The pharmaceutical composition according to any one of (1) to (6), wherein the sustained-release layer b) comprises 25 to 45 wt.% of the sustained-release agent, preferably 30 to 42 wt.% and more preferably 32 to 37 wt.% such as 32 wt.% of the sustained-release agent, based on the total weight of the sustained-release layer b).
(8) The pharmaceutical composition according to any one of (1) to (7), wherein, in a matrix of the sustained-release layer b), the sustained-release agent is preferably selected form hydroxypropylmethylcellulose (HPMC) or hydroxyethylcellulose (HEC) grades with a viscosity in a range 2 700 - 5 500, more preferably 2500 - 5 040 mPas, most preferably 4 000 mPas measured according to EU Pharmacopoeia 7.0 method 2.2.8 in 2% water solution.
(9) The pharmaceutical composition according to (7) or (8), wherein the hydroxypropylmethylcellulose (HPMC) or matrix is formed by a HPMC grade K4M or E4M and the Hydroxyethylcellulose (HEC) matrix is formed by a HEC grade HHX or any combination thereof. Preferably the hydroxypropylmethylcellulose (HPMC) matrix is formed by a HPMC grade K4M or HEC grade HHX. Most preferably the matrix is formed by a HPMC grade K4M.
(10) The pharmaceutical composition according to (9) wherein the HPMC matrix is a combination of hydroxypropylmethylcelluloses K4M and E4M or combination of hydroxypropylmethylcellulose and hydroxyethylcellulose, such as K4M and HHX or E4M and HHX.
(11) The pharmaceutical composition according to (11) or (12), wherein the sustained release agents being a hydroxypropylmethylcellulose (HPMC) or hydroxyethylcellulose (HEC) are used from 2:8 to 1:1, preferably 1:1.
(12) The pharmaceutical composition according to any one of (1) to (11), wherein the immediate release layer a) comprises, as the at least one excipient, at least one of a filler, a disintegrant, a binder, a lubricant, a glidant and any combination thereof. More preferably, the immediate release layer a) contains a binder in addition to the filler, the disintegrant, and the lubricant. More preferably, the immediate-release layer b) contains no glidant.
(13) The pharmaceutical composition according to (1) to (12), wherein the sustained-release layer b) comprises, as the at least one excipient, at least one of a filler, a binder, a lubricant, a glidant, a disintegrant and any combination thereof. Preferably, the sustained-release layer b) comprises as the at least one excipient a filler, a binder, a lubricant, and a glidant.
(14) The pharmaceutical composition according to any one of (1) to (13), wherein the immediate release layer a) and/or the sustained-release layer b) comprise a filler. Preferably, the immediate release layer a) and sustained-release layer b) comprise a filler.
(15) The pharmaceutical composition according to (14), wherein the filler is at least one independently selected from the group consisting of anhydrous or monohydrate lactose, sucrose, microcrystalline cellulose, starch such as maize starch, pregelatinized starch, microcrystalline cellulose coated with colloidal silica, mannitol and any combination thereof, preferably the filler is at least one independently selected from the group consisting of anhydrous or monohydrate lactose, mannitol, microcrystalline cellulose, maize starch, saccharose and any combination thereof, and more preferably the filler is at least one selected from the group consisting of lactose monohydrate, microcrystalline cellulose, or a combination thereof.
(16) The pharmaceutical composition according to (14) or (15), wherein the immediate release layer a) contains the filler in an amount of 80 to 90 wt.%, preferably 85 to 87 wt.%, based on the weight of the immediate release layer a), and/or wherein the sustained-release layer b) contains the filler in an amount of 50 to 70 wt.%, preferably 52 to 69 wt.%, more preferably 57 to 63 wt.% based on the weight of the sustained-release layer b).
(17) The pharmaceutical composition according to any one of (1) to (16), wherein the immediate release layer a) and/or the sustained-release layer b) comprise a binder. Preferably, the immediate release layer a) and the sustained-release layer b) comprise a binder.
(18) The pharmaceutical composition according to (17), wherein the binder is at least one independently selected from the group consisting of hydroxypropylcellulose, hydroxypropylmethylcellulose, methylcellulose, carboxymethylcellulose, hydroxycellulose, hydroxyethylcellulose, polyvinyl alcohol, polyvinylpyrrolidone, copovidone, pregelatinized starch, gelatine and any combination thereof, preferably the binder is at least one independently selected from the group consisting of hydroxypropyl cellulose, polyvinylpyrrolidone and any combination thereof, and more preferably the binder is polyvinylpyrrolidone or polyvinyl alcohol.
(19) The pharmaceutical composition according to (17) or (18), wherein the immediate release layer a) contains the binder in an amount of 1 to 3 wt.%, preferably 1.5 to 2 wt.%, such as 1.75 wt.% based on the weight of the immediate release layer a) and/or wherein the sustained-release layer b) contains the binder in an amount of 4 to 5 wt.%, preferably 4.2 to 4.5 wt.%, such as 4.3 wt.% based on the weight of the sustained-release layer b). Preferably the wt.% ratio of ramipril to polyvinyl alcohol is in range of 1.1 to 4.0, preferably 1.15 to 3.9, more preferably 1.7 to 2.85, such as 1.75 or 2.8.
(20) The pharmaceutical composition according to any one of (1) to (19), wherein the immediate release layer a) and/or the sustained-release layer b) comprise a disintegrant. Preferably, the immediate release layer a) comprises a disintegrant.
(21) The pharmaceutical composition according to (20), wherein the disintegrant is at least one independently selected from the group consisting of carboxymethylcellulose sodium salt, sodium croscarmellose, microcrystalline cellulose, crospovidone, sodium starch glycolate, maize starch, and any combination thereof, preferably the disintegrant is at least one independently selected from the group consisting of sodium starch glycolate, sodium croscarmellose, maize starch and any combination thereof, more preferably the disintegrant is sodium croscarmellose or sodium starch glycolate.
(22) The pharmaceutical composition according to (20) or (21), wherein the immediate release layer a) contains the disintegrant in an amount of 3 to 7 wt.%, preferably 4 to 6 wt.%, more preferably 5 wt.%, based on the weight of the immediate release layer a).
(23) The pharmaceutical composition according to any one of (1) to (22), wherein the immediate release layer a) and/or the sustained-release layer b) comprise a lubricant. Preferably, the immediate release layer a) and the sustained-release layer b) comprise a lubricant.
(24) The pharmaceutical composition according to (23), wherein the immediate release layer a) contains the lubricant in an amount of 0.5 to 3 wt.%, preferably 1 to 2.5 wt.%, such as 2 wt.% based on the weight of the immediate release layer a), and/or wherein the sustained-release layer b) contains the lubricant in an amount of 0.2 to 0.6 wt.%, preferably 0.3 to 0.6 wt.%, such as 0.6 wt.% based on the weight of the sustained-release layer b).
(25) The pharmaceutical composition according to (24), wherein the lubricant is at least one independently selected from the group consisting of stearic acid, magnesium stearate, calcium stearate, sodium stearyl fumarate, glycerol tristearate, palm oil derivatives such as palmitic acid or hydrogenated palm oil, hydrogenated vegetable oil, such as hydrogenated castor oil, and any combination thereof, preferably the lubricant is at least one independently selected from magnesium stearate and stearic acid.
(26) The pharmaceutical composition according to any one of (1) to (25), wherein the immediate release layer a) and/or the sustained-release layer b) comprise a glidant. Preferably, the sustained-release layer b) but not the immediate release layer a) comprises a glidant.
(27) The pharmaceutical composition according to (26), wherein the glidant is at least one independently selected from the group consisting of fumed silica (colloidal silicon dioxide), such as colloidal anhydrous silica, starch, talc and any combination thereof, preferably the glidant is colloidal anhydrous silica.
(28) The pharmaceutical composition according to (26) or (27), wherein the sustained-release layer b) contains the glidant in an amount of up to 1 wt.%, based on the weight of the sustained-release layer b), and preferably 0.1 to 0.5 wt.%, 0.2 to 0.03 wt.%, such as 0.2 wt.% based on the weight of the sustained-release layer b).
(29) The pharmaceutical composition according to any one of (1) to (28), wherein the immediate release layer a) comprises 80 to 90 wt.%, preferably 85 to 87 wt.%, of a filler 3 to 7 wt.%, preferably 4 to 6 wt.%, such as 5 wt.% of a disintegrant, 0.5 to 3 wt.%, preferably 1 to 2.5 wt.%, such as 2 wt.%, of a lubricant based on the total weight of the immediate release layer a).
(30) The pharmaceutical composition according to any one of (1) to (29), wherein the immediate release layer a) comprises 0.75 to 5 wt.%, preferably 1.5 to 4.5 wt.%, more preferably 1.75 to 3.75% wt.%, preferably 0.75, 1.75,1.25, 2.25, 2.75, 3.25, 3.75, 4.25, 4.5, 4.77 wt.% of a binder, most preferably 1.75 based on the total weight of the immediate release layer a) and the sustained release layer b) comprises 4 to 5 wt.%, preferably 4.2 to 4.5 wt.%, more preferably 4.3 wt.%, of a binder, based on the total weight of the sustained release layer b).
(31) The pharmaceutical composition according to any one of (1) to (30), wherein the sustained-release layer b) comprises 25 to 45 wt.%, preferably 30 to 42 wt.%, more preferably 32 to 37 wt.%, of the sustained-release agent, 50 to 70 wt.%, preferably 52 to 69 wt.%, more preferably 57 to 63 wt.% of a filler, 4 to 5 wt.%, preferably 4.2 to 4.5 wt.%, such as 4.3 wt.% of a binder, 0.2 to 0.6 wt.%, preferably 0.3 to 0.55 wt.%, such as 0.5 wt.% of a lubricant, and, 0.1 to 0.5 wt.%, 0.2 to 0.3 wt.%, such as 0.2 wt.% of a glidant.
(32) The pharmaceutical composition according to any one of (1) to (31), wherein the immediate release layer a) contains 4.9 wt.% of ramipril, 86,35 wt.% of Lactose monohydrate, 5.0 wt.% of Croscarmellose sodium, 1.75 wt.% of Polyvinyl alcohol and 2 wt.% sodium stearyl fumarate, based on the total weight of the immediate release layer a).
(33) The pharmaceutical composition according to any one of (1) to (32), wherein the sustained-release layer b) contains 0.75 wt.% of indapamide, 57.25 to 62.25 wt.% of lactose monohydrate, 32 to 37 wt.% of hydroxypropylmethylcellulose K4M or E4M or hydroxyethylcellulose HHX, 4.30 wt.% of polyvinylpyrrolidone, 0.2 wt.% of colloidal anhydrous silica, and 0.5 wt.% of magnesium stearate, based on the total weight of the sustained release layer b).
(34) The pharmaceutical composition according to any one of (9) to (33), wherein, the work parameter value for the matrix of the sustained-release layer b) is in a swollen state is below 38 -37 N*mm, wherein the swollen state is obtained by immersing the dry sustained release layer b) in a 30 to 35 mL of a medium: pH=1.2 (0.1M aqueous HCl solution) for 1 hour and subsequently in a 30 to 35 mL of a medium phosphate buffer pH=4,5 for 1 h, contained in beaker the beaker being placed in a wiggled, heated bath at 37°C. Such a matrix of the sustained-release layer b) is in a swollen state and has a thickness in the range of range of 6.5-8.0 mm, preferably 6.7 -7.3 mm
(35) The pharmaceutical composition according to any one of (1) to (34), wherein the pharmaceutical composition has dissolution release rate of indapamide of 10 to 30 wt.%, preferably 12 to 19 wt.% of indapamide within 2 h, 40 to 65 wt.% within 8 h, 45 to 65% within 10h and above 85 wt.% within 21 h, based on the total indapamide content as determined by using European Pharmacopoeia edition 8 Apparatus Paddles "2" in 900 mL volume of Phosphate buffer pH 6.8 at 37.0°C±0.5°C and 50 rpm, and dissolution rate of ramipril of 85 wt.%, preferably 90 wt.%, most preferably 97wt.% of ramipril within 15 minutes, based on the total ramipril content as determined by using European Pharmacopoeia edition 8 Apparatus Baskets "1" in 500 mL volume of 0.1 M HCl (pH 1.2) at 37.0°C±0.5°C and 100 rpm.

According to a preferred aspect of the present invention, the fixed dose pharmaceutical composition for oral administration has the features according to the above (1) to (2). Thus, a the prolonged-release pharmaceutical composition in form of a tablet for oral administration is provided, which comprises: a) an immediate release layer comprising Ramipril and polyvinyl alcohol in a wt.% ratio of 1.1 to 4.0, such as 2.8. and one or more pharmaceutically acceptable excipients; and b) a sustained-release layer, comprising Indapamid, a sustained-release agent selected from of hydroxypropylmethylcellulose (HPMC) or hydroxyethylcellulose (HEC) grades with a viscosity in a range of 2 500 - 5 500 mPas, measured according to EU Pharmacopoeia 7.0 method 2.2.8 in 2% water solution, and one or more pharmaceutically acceptable excipients, in which the immediate release layer a) forms a first layer, the sustained-release layer b) forms a second layer. The pharmaceutical composition for oral administration according to this aspect may be further characterized according to any one of the above (3) and to (35). According to a further aspect, the said pharmaceutical composition is for use in a method of treatment of a hypertension.

According to a preferred aspect of the present invention, the fixed dose pharmaceutical composition for oral administration has the features according to the above (1) and (3). Thus, a the prolonged-release pharmaceutical composition in form of a tablet for oral administration is provided, which comprises: a) an immediate release layer comprising Ramipril and polyvinyl alcohol in a wt.% ratio of 1.1 to 4.0, such as 2.8. and one or more pharmaceutically acceptable excipients; and b) a sustained-release layer, comprising Indapamid, a sustained-release agent selected from of hydroxypropylmethylcellulose (HPMC) or hydroxyethylcellulose (HEC) grades with a viscosity in a range of 2 500 - 5 500 mPas, measured according to EU Pharmacopoeia 7.0 method 2.2.8 in 2% water solution, and one or more pharmaceutically acceptable excipients, in which in the immediate release layer a) the wt.% ratio of ramipril to polyvinyl alcohol is in a range of 1.1 to 4.0, preferably 1.15 to 3.9, more preferably 1.7 to 2.85, such as 1.75 or 2.8.

According to a further preferred aspect of the present invention, the pharmaceutical composition for oral administration has the features of the above (1) and (5). Thus, a pharmaceutical composition in form of a bilayer tablet for oral administration is provided, which comprises: a) an immediate release layer comprising Ramipril and polyvinyl alcohol in a wt.% ratio of 1.1 to 4.0, such as 2.8 and one or more pharmaceutically acceptable excipients; and b) a sustained-release layer, comprising Indapamid, a sustained-release agent selected from of hydroxypropylmethylcellulose (HPMC) or hydroxyethylcellulose (HEC) grades with a viscosity in a range of 2 500 - 5 500 mPas, measured according to EU Pharmacopoeia 7.0 method 2.2.8 in 2% water solution, and one or more pharmaceutically acceptable excipients, wherein the immediate release layer a) comprises 10, 5 or 2.5 mg of ramipril calculated on a ramipril free acid, and the sustained-release layer b) comprises 1.5 mg of indapamid; and, preferably, the immediate release layer a) comprises 10 or 5 mg of ramipril, and the sustained-release layer b) comprises 1.5 mg of indapamid. The pharmaceutical composition for oral administration according to this aspect may be further characterized according to any one of the above (2) and (4) to (35).

According to a further preferred aspect of the present invention, the pharmaceutical composition for oral administration has the features of the above (1) and (6). Thus, a pharmaceutical composition in form of a bilayer tablet for oral administration is provided, which comprises: a) an immediate release layer comprising Ramipril and polyvinyl alcohol in a wt.% ratio of 1.1 to 4.0, such as 2.8 and one or more pharmaceutically acceptable excipients; and b) a sustained-release layer, comprising Indapamid, a sustained-release agent selected from of hydroxypropylmethylcellulose (HPMC) or hydroxyethylcellulose (HEC) grades with a viscosity in a range of 2 500 - 5 500 mPas, measured according to EU Pharmacopoeia 7.0 method 2.2.8 in 2% water solution, and one or more pharmaceutically acceptable excipients, and the immediate release layer a) has a ramipril content of 4.5 to 5.5 wt.%, preferably 4.5 to 5.0 wt.%, more preferably 4.5 to 4.9 wt.%, such as 4.9 wt.%, based on the total weight of the immediate release layer a); furthermore, the sustained release layer b) has a indapamide content of 0.5 to 1.0 wt.%, preferably 0.6 to 0.9 wt.%, more preferably 0.6 to 0.8 wt.%, such as 0.75 wt.%, based on the total weight of the sustained-release layer b).

According to a further preferred aspect of the present invention, the pharmaceutical composition for oral administration has the features of the above (1) and (29).Thus, a pharmaceutical composition in form of a bilayer tablet for oral administration is provided, which comprises an immediate release layer comprising Ramipril and polyvinyl alcohol in a wt.% ratio of 1.1 to 4.0, such as 2.8 and one or more pharmaceutically acceptable excipients; and b) a sustained-release layer, comprising Indapamid, a sustained-release agent selected from of hydroxypropylmethylcellulose (HPMC) or hydroxyethylcellulose (HEC) grades with a viscosity in a range of 2 500 - 5 500 mPas, measured according to EU Pharmacopoeia 7.0 method 2.2.8 in 2% water solution, and one or more pharmaceutically acceptable excipients and the immediate release layer a) comprising 80 to 90 wt.%, preferably 85 to 87 wt.%, of a filler 3 to 7 wt.%, preferably 4 to 6 wt.%, such as 5 wt.% of a disintegrant, 0.5 to 3 wt.%, preferably 1 to 2.5 wt.%, such as 2 wt.%, of a lubricant based on the total weight of the immediate release layer a) and

According to a further preferred aspect of the present invention, the pharmaceutical composition for oral administration has the features of the above (1) and (30).Thus, a pharmaceutical composition in form of a bilayer tablet for oral administration is provided, which comprises an immediate release layer comprising Ramipril and polyvinyl alcohol in a wt.% ratio of 1.1 to 4.0, such as 2.8 and one or more pharmaceutically acceptable excipients; and b) a sustained-release layer, comprising Indapamid, a sustained-release agent selected from of hydroxypropylmethylcellulose (HPMC) or hydroxyethylcellulose (HEC) grades with a viscosity in a range of 2 500 - 5 500 mPas, measured according to EU Pharmacopoeia 7.0 method 2.2.8 in 2% water solution, and one or more pharmaceutically acceptable excipients and the immediate release layer a) comprises 0.75 to 5 wt.%, preferably 1.5 to 4.5 wt.%, more preferably 1.75 to 3.75% wt.%, preferably 0.75, 1.75,1.25, 2.25, 2.75, 3.25, 3.75, 4.25, 4.5, 4.77 wt.% of a binder, most preferably 1.75 based on the total weight of the immediate release layer a) and the sustained release layer b) comprises 4 to 5 wt.%, preferably 4.2 to 4.5 wt.%, more preferably 4.3 wt.%, of a binder, based on the total weight of the sustained release layer b).

According to a further preferred aspect of the present invention, the pharmaceutical composition for oral administration has the features of the above (32) and (33). Thus, a pharmaceutical composition in form of a bilayer tablet for oral administration is provided, which comprises: a) the immediate release layer a) which contains 4.9 wt.% of ramipril, 86,35 wt.% of Lactose monohydrate, 5.0 wt.% of Croscarmellose sodium, 1.75 wt.% of Polyvinyl alcohol and 2 wt.% sodium stearyl fumarate, based on the total weight of the immediate release layer a) and the sustained-release layer b) which contains 0.75 wt.% of indapamide, 57.25 to 62.25 wt.% of lactose monohydrate, 32 to 37 wt.% of hydroxypropylmethylcellulose K4M or E4M or hydroxyethylcellulose HHX, 4.30 wt.% of polyvinylpyrrolidone, 0.2 wt.% of colloidal anhydrous silica, and 0.5 wt.% of magnesium stearate, based on the total weight of the sustained release layer b).

In most preferred aspect a pharmaceutical composition in form of a bilayer tablet for oral administration is provided, which comprises: a) an immediate release layer comprising 4.9% of ramipril, 86.35 wt.% of Lactose monohydrate, 5 wt.% of Croscarmellose sodium, 1.75 wt.% Polyvinyl alcohol and 2 wt.% of Stearyl fumarate sodium, based on the total weight of the immediate release layer a) and 0.75 wt.% of indapamide, 62.25 wt. % of lactose monohydrate, 4,30 wt. % of polyvinylpyrrolidone, 32,00 wt.% of Hypromellose K4M, 0.20 wt.% of colloidal anhydrous silica, and 0.50 wt.% of magnesium stearate based on the total weight of the sustained release layer b).

In such most preferred aspect the composition comprises:
in the sustained release layer b) 1.5 mg of indapamide, 124.5 mg of lactose monohydrate, 8.6 mg of povidone K-30, 64 mg of Hypromellose K4M, 0.4 mg of Silica, Colloidal Anhydrous, 1 mg of Magnesium Stearate and in the immediate release layer a) 10 mg of Ramipril, 176.15 mg of Lactose monohydrate, 10.2 mg of Croscarmellose sodium, 3.57 mg of Polyvinyl alcohol, and 4.08 mg of Stearyl fumarate sodium; or comprises:
in the sustained release layer b) 1.5 mg of indapamide, 124.5 mg of lactose monohydrate, 8.6 mg of povidone K-30, 64 mg of Hypromellose K4M, 0.4 mg of Silica, Colloidal Anhydrous, 1 mg of Magnesium Stearate and in the immediate release layer a) 5 mg of Ramipril, 88.08 mg of Lactose monohydrate, 5.1 mg of Croscarmellose sodium, 1.78 mg of Polyvinyl alcohol, and 2.04 mg of Stearyl fumarate sodium.

Fixed dose bilayer tablet according to the present invention provides patients with an effective, safe and convenient hypertension therapy when concurrent administration of ramipril and indapamide is required.

Patients using such a therapy would find it easier to take the drug regularly as scheduled, leading to an increase of the compliance. The tablet providing immediate release of ramipril and sustained release of indapamide ensures maintaining a therapeutic level of the active substances over the time and according to the requirements set for the referents of the both actives, respectively. This is beneficial from both the clinical and the patient's perspective, since there is no need for compliance control of administration of subsequent drug doses. Appropriate dissolution profiles and stabilities of both actives in the bilayer tablet according to the invention are achieved, and by consequence, suitability for use in a medical treatment.

### Detailed description

According to an aspect of the present invention, a pharmaceutical composition for oral administration in form of bilayer tablet is provided, the pharmaceutical composition comprising: a) an immediate release layer, comprising ramipril and polyvinyl alcohol in a wt.% ratio in a range of 1.1 to 4.0 and one or more pharmaceutically acceptable excipients; and b) a sustained-release layer, comprising indapamide, a sustained-release agent being hydroxypropylmethylcellulose or hydroxyethylcellulose with a viscosity in a range of 2 500 - 5 500 mPas, measured according to EU Pharmacopoeia 7.0 method 2.2.8 in 2% water solution, and one or more pharmaceutically acceptable excipients.

An immediate release (IR) layer releases ramipril with no special rate controlling features, whereas the sustained-release (SR) layer releases indapamide gradually within 24 hours owing to the sustained-release agent. The understanding of the terms correspond to common pharmaceutical handbook definitions. The immediate release layer shows a release of the active substance, which is not deliberately modified by a special formulation and/or manufacturing method. The immediate-release (IR) layer ensures that ramipril is available to the body without relevant impact of the dosage form. In particular, according to the present invention, the IR layer achieves in vitro dissolution of at least 85 wt.% of the ramipril within 15 minutes, based on the total ramipril content of the IR layer. The sustained-release layer releases the active ingredient at a sustained rate, in particular, a constant rate. Preferably, the release rate is independent from the pH, the ionic content, and other contents within the entire segment of the gastrointestinal tract, in particular the stomach and the small intestine. In particular, according to the present invention, the sustained-release layer has an in vitro dissolution rate of 10 to 30 wt.% of indapamide within 2 h, based on the total indapamide content of the SR layer. The 2-h time point of the dissolution profile has been chosen as representative for the correlation and referent for the prediction of the required full dissolution profile (up to release of the total amount of the API). The aforementioned in vitro dissolution rates are determined for indapamide by using European Pharmacopoeia edition 8 Apparatus Paddles "2" in 900 mL volume of Phosphate buffer pH 6.8 at 37.0°C±0.5°C and 50 rpm, and for ramipril by using European Pharmacopoeia edition 8 Apparatus Baskets "1" in 500 mL volume of 0.1 M HCl (pH 1.2) at 37.0°C±0.5°C and 100 rpm.

The combination of IR layer and SR layer in form of a bilayer tablet according to the present invention provides a sustained release of indapamide comparable to that of prior art a referent indapamide sustained release oral dosage form and an immediate release of ramipril compared to that of prior art immediate release referent immediate release ramipril dosage form, both administered by the same oral route. In particular, the fixed dose pharmaceutical composition achieves in vitro dissolution rate of indapamide of 10 to 30 wt.%, preferably 12 to 19wt.% of indapamide within 2 h, 40 to 65 wt.% within 8 h, 45 to 65wt.% within 10h and above 85 wt.% within 21 h, based on the total indapamide content as determined by using European Pharmacopoeia edition 8 Apparatus Paddles "2" in 900 mL volume of Phosphate buffer pH 6.8 at 37.0°C±0.5°C and 50 rpm, and dissolution rate of ramipril of 85 wt.%, preferably 90wt.%, most preferably 97wt.% of ramipril within 15 minutes, based on the total ramipril content as determined by using European Pharmacopoeia edition 8 Apparatus Baskets "1" in 500 mL volume of 0.1 M HCl (pH 1.2) at 37.0°C±0.5°C and 100 rpm.

In the pharmaceutical composition, ramipril is preferably contained as the free acid and indapamide is contained as a free amide.

According to an embodiment, the pharmaceutical composition is a single dosage form in the form of a tablet. In the tablet, the immediate release layer a) forms a first layer, the sustained-release layer b) forms a second layer, and the first layer at least partially covers the surface of the second layer. The second layer may be completely surrounded by the first. However, it is preferable that the tablet is a bilayer tablet, which means that the first layer is arranged on one side (i.e. one of the two faces) of the second layer and, typically, covers this side completely. Such a tablet can be produced economically.

The tablet may be uncoated or coated. The coating may, for example, be a film-coating. The tablet may have any suitable coating, e.g. functional coating. Such coatings can be found in the "Pharmaceutical Manufacturing Handbook" edited by Shayne Cox Gad, published by John Wiley & Sons, Inc., Hoboken, New Jersey, March 2008. Preferably, the tablet is coated. The coating has an aesthetic function and is not a functional coating, i.e. the coating doesn't influence on dissolution rate of the active substances form their respective layers. A film coat comprising HPMC, Macrogol 6000 and a (dye) coat may optionally applied on the tablet core, such as Colorcon Opadry^{®}. Preferred is the use of Opadry^{®} complete film coating system 03F180012 with water as the solvent. Most preferable is Colorcon Opadry^{®} II complete film coating system 85F18422.

In the pharmaceutical composition, the IR layer a) and the SR layer b) may be compressed together, for example, by using a bilayer-tableting machine. The components of IR layer and the components of SR layer may also be granulated and then be compressed to a tablet, for example, by using a bilayer-tableting machine. Preferably, in the pharmaceutical composition, the immediate release layer a) is compressed from granulates, and the sustained-release layer b) is compressed from granulates,. The granulates may be obtained by common dry or wet granulation techniques, preferably by wet granulation. In addition to granulates, before being compressed to the respective the immediate release layer a) and/or the sustained-release layer b) may also contain an extragranular fraction, for example, comprising one or more of a lubricant, a filler, an absorbent and a glidant or sustained release agent. The components of an extragranular fraction are added after formation of the granulates, blended and then compressed to the respective layers.

According to an embodiment, in the pharmaceutical composition, the immediate release layer a) may contain 2.5 to 10 mg of ramipril, and the sustained-release layer b) may contain 1.5 to 2.5 mg of indapamide, preferably 1.5 mg of indapamide. Preferably, the immediate release layer a) contains 5 or 10 mg of ramipril, and the sustained-release layer b) contains 1.5 mg of indapamide.

In the aforementioned embodiment, the immediate release layer a) has a ramipril content of 4.5 to 5.5 wt.%, preferably 4.5 to 5.0 wt.%, more preferably 4.5 to 4.9 wt.%, such as 4.9 wt.%, based on the total weight of the immediate release layer a); furthermore, the sustained release layer b) has a indapamide content of 0.5 to 1.0 wt.%, preferably 0.6 to 0.9 wt.%, more preferably 0.6 to 0.8 wt.%, such as 0.75 wt.%, based on the total weight of the sustained-release layer b).

In the pharmaceutical composition, the sustained-release layer b) contains a sustained-release agent in order to modify and sustain the release of indapamide from the SR layer. Due to the sustained-release agent, the SR layer has the above in vitro release values. The immediate release layer a) has no modified or sustained release of ramipril as noted above. In the pharmaceutical composition, the sustained-release agent may be used in an amount of 25 to 45 wt.% of the sustained-release agent, preferably 30 to 42 wt.% and more preferably 32 to 37 wt.% such as 32 wt.% of the sustained-release agent, based on the total weight of the sustained-release layer b).

The , sustained-release agent is contained in a matrix of the sustained-release layer b). The matrix of the sustained-release layer b) may be formed by the sustained-release agent or the sustained-release agent and a binder. According to the claimed invention, the sustained-release agent is at least one of hydroxypropylmethylcellulose (HPMC) or hydroxyethylcellulose (HEC) grades with a viscosity in a range of 2 500 - 5 500, preferably 2 700 - 5 500, most preferably 2500 - 5 040 mPas, such as 4 000 mPas measured according to EU Pharmacopoeia 7.0 method 2.2.8 in 2wt.% water solution. Preferably, the sustained release agent is a HPMC grade K4M or E4M or a HEC grade HHX. In one preferred embodiment a mixture of at least two of HPMC grades K4M or E4M and a HEC grade HHX is used in a ratio from 2:8 to 1:1. Hence, the matrix of the SR layer is preferably formed by the HPMC grade K4M or E4M or a mixture thereof or the HPMC grade K4M or E4M or a mixture thereof and a binder. Also preferably, the matrix of the SR layer is formed by one of the HPMC grades K4M, E4M in a mixture with HEC grade HHX , optionally with a binder. In the SR layer, indapamide is preferably dispersed or suspended in the matrix, such that the release is sustained.

More preferably, a thickness of the matrix of the sustained release layer in a swollen is in a range of 6.5-8.0 mm, preferably 6.7 -7.3 mm, and for such a matrix the work parameter was below 38 -37 N*mm, wherein the swollen state is obtained by immersing the dry sustained release layer b) in a 30 to 35 mL of a medium: pH=1.2 (0.1M aqueous HCl solution) for 1 hour and subsequently in a 30 to 35 mL of a medium phosphate buffer pH=4,5 for 1 h, contained in beaker the beaker being placed in a wiggled, heated bath at 37°C.

Also, combinations of two or more hydroxypropylmethylcelluloses and a hydroxyethylcellulose grade HHX are preferred. Such as combination of HPMC grades K4M or E4M with HEC grade HHX. Preferably combination of K4M and HHX or E4M and HHX is used is used in a ratio from 2:8 to 1:1. Most preferably, hydroxypropylmethylcellulose K4M is used with Hydroxyethylcellulose HHX.

In the pharmaceutical composition, the immediate release layer a) and the sustained-release layer b) each comprise also at least one excipient. As the at least one excipient, the immediate release layer a) may comprise at least one of a filler, a disintegrant, a binder, a lubricant and any combination thereof. Preferably, the immediate release layer a) comprises a filler, a disintegrant, a binder, a lubricant, and, optionally a glidant. The immediate release layer a) preferably consists of ramipril and a filler, a disintegrant, a lubricant, and a binder.

The sustained-release layer b) may comprise, as the at least one excipient, at least one of a filler, a binder, a lubricant, a glidant and any combination thereof, preferably the sustained-release layer b) comprises a filler, a disintegrant, a binder, a lubricant, and, optionally, a glidant. The sustained-release layer b) preferably consists of indapamide, a sustained-release agent, a filler, a binder, a lubricant, and a glidant. Preferably, the SR layer does not contain a disintegrant.

In the pharmaceutical composition, the immediate release layer a) and/or, preferably and, the prolonged-release layer b) comprise a filler. Herein, a filler means at least one filler. Accordingly, the filler may be at least one filler independently selected from the group consisting of anhydrous or monohydrate lactose, sucrose, microcrystalline cellulose, starch such as maize starch, pregelatinized starch, microcrystalline cellulose coated with colloidal silica, mannitol and any combination thereof. Preferably, the filler is at least one independently selected from the group consisting of anhydrous or monohydrate lactose, mannitol, microcrystalline cellulose, maize starch, saccharose and any combination thereof. More preferably, the filler is at least one selected from the group consisting of lactose monohydrate, microcrystalline cellulose, or a combination thereof. The immediate release layer a) contains the filler in an amount of 80 to 90 wt.%, preferably 85 to 87 wt.%, based on the weight of the immediate release layer a), and/or wherein the sustained-release layer b) contains the filler in an amount of 50 to 70 wt.%, preferably 52 to 69 wt.%, more preferably 57 to 63 wt.% based on the weight of the sustained-release layer b).

In the pharmaceutical composition, the immediate release layer a) and/or the sustained-release layer b) comprise a binder. Preferably the immediate release layer a) and the sustained-release layer b) comprise a binder, the binder being contained in addition to the sustained-release agent. Herein, a binder means at least one binder. Accordingly, the binder may, for example, be at least one binder independently selected from the group consisting of hydroxypropylcellulose, hydroxypropylmethylcellulose, methylcellulose, carboxymethylcellulose, hydroxycellulose, hydroxyethylcellulose, polyvinyl alcohol, polyvinylpyrrolidone, copovidone, pregelatinized starch, gelatine and any combination thereof. Preferably, the binder is at least one independently selected from the group consisting of hydroxypropyl cellulose, polyvinylpyrrolidone and any combination thereof. More preferably the binder is polyvinylpyrrolidone and polyvinyl alcohol. The immediate release layer a) comprises 0.75 to 5 wt.%, preferably 1.5 to 4.5 wt.%, more preferably 1.75 to 3.75% wt.%, preferably 0.75, 1.75,1.25, 2.25, 2.75, 3.25, 3.75, 4.25, 4.5, 4.77 wt.% of a binder, most preferably 1.75 based on the total weight of the immediate release layer a) and/or wherein the sustained-release layer b) contains the binder in an amount of 4 to 5 wt.%, preferably 4.2 to 4.5 wt.%, such as 4.3 wt.% based on the weight of the sustained-release layer b). Most preferably immediate release layer a) contains the polyvinyl alcohol as a binder. According to the claimed invention, the wt.% ratio of ramipril to polyvinyl alcohol is in range of 1.1 to 4.0, preferably 1.15 to 3.9, more preferably 1.7 to 2.8, such as 2.8. A binder is not required if the respective component is produced by dry granulation or direct compacting. It is however preferred to use wet granulation in the preparation of the components of the layers, where a binder is commonly used as described in more detail below.

In the pharmaceutical composition, the immediate release layer a) and/or the sustained-release layer b) comprise a disintegrant. Herein, a disintegrant means at least one disintegrant. Accordingly, the disintegrant may, for example, be at least one disintegrant independently selected from the group consisting of carboxymethylcellulose sodium salt, sodium croscarmellose, microcrystalline cellulose, crospovidone, sodium starch glycolate, maize starch, and any combination thereof. Preferably, the disintegrant is at least one independently selected from the group consisting of sodium starch glycolate, sodium croscarmellose, maize starch and any combination thereof. More preferably, the disintegrant is sodium croscarmellose or sodium starch glycolate. The disintegrant may be used in an amount of 3 to 7 wt.%, preferably 4 to 6 wt.%, more preferably 5 wt.%, based on the total mass of the respective layer. Preferably only the immediate release layer a) may contain the disintegrant in an amount of 3 to 7 wt.%, preferably 4 to 6 wt.%, more preferably 5 wt.%, based on the weight of the immediate release layer a).

In the pharmaceutical composition, the immediate release layer a) and/or, preferably and, the sustained-release layer b) comprise a lubricant. Herein, a lubricant means at least one lubricant. Accordingly, the lubricant may, for example, be at least one lubricant independently selected from the group consisting of stearic acid, magnesium stearate, calcium stearate, sodium stearyl fumarate, glycerol tristearate, palm oil derivatives such as palmitic acid or hydrogenated palm oil, especially Softisan 154, hydrogenated vegetable oil, such as hydrogenated castor oil, and any combination thereof. Preferably, the lubricant is at least one independently selected from magnesium stearate and stearic acid. The immediate release layer a) may contain the lubricant in an amount of 0.5 to 3 wt.%, preferably 1 to 2.5 wt.%, such as 2 wt.% based on the weight of the immediate release layer a). The sustained-release layer b) may contain the lubricant in an amount of 0.2 to 0.6 wt.%, preferably 0.3-0.5wt.%, such as 0.5 wt.%, based on the weight of the sustained-release layer b).

In the pharmaceutical composition, the immediate release layer a) and/or the sustained-release layer b) may comprise a glidant. It is preferred that the sustained release layer b) but not the immediate-release layer a) comprises a glidant. Herein, a glidant means at least one glidant. Accordingly, the glidant may, for example, be at least one glidant independently selected from the group consisting of fumed silica (colloidal silicon dioxide), such as colloidal anhydrous silica, starch, talc and any combination thereof. Preferably, the glidant is colloidal anhydrous silica. The glidant may be used in an amount of 0.1 to 0.5 wt.%, preferably 0.2 to 0.3 wt.%, more preferably 0.2 wt.%, based on the total mass of the composition.

Particularly preferred pharmaceutical compositions of the present invention are obtained by combining preferred embodiments such as preferred contents and preferred components.

According to a particularly preferred embodiment, the pharmaceutical composition is a single dosage form in the form of a bilayer tablet, in which the immediate release layer a) forms a first layer, the sustained-release layer b) forms a second layer. The immediate release layer a) comprises 10, 5 or 2.5 mg of ramipril calculated on a ramipril free acid, and the sustained-release layer b) comprises 1.5 mg of indapamid; and, preferably, the immediate release layer a) comprises 10 or 5 mg of ramipril, and the sustained-release layer b) comprises 1.5 mg of indapamid.

In the matrix of the sustained-release layer b), the sustained-release layer b) comprises 25 to 45 wt.%, preferably 30 to 42 wt.%, more preferably 32 to 37 wt.%, of a HPMC or HEC as the sustained-release agent, the HPMC or HEC being at least one selected from the group of grades with a viscosity in a range of 2 500 - 5 500, preferably 2 700 - 5 500, most preferably 2500 - 5 040 mPas, such as 4 000 mPas measured according to EU Pharmacopoeia 7.0 method 2.2.8 in 2wt.% water solution and any combination thereof.

According to another particularly preferred embodiment, the immediate release layer a) comprises 80-90 wt.%, preferably 85-87 wt.%, of a filler, 0.5-3.0 wt.%, preferably 1-2.5 wt.%, most preferably 2.0 wt.%, of a lubricant and 0.75 to 5 wt.%, preferably 1.5 to 4.5 wt.%, more preferably 1.75 to 3.75% wt.%, preferably 0.75, 1.75,1.25, 2.25, 2.75, 3.25, 3.75, 4.25, 4.5, 4.77 wt.% of a binder, most preferably 1.75, based on the total weight of the immediate release layer a).

The immediate release layer a) may comprise 0.75 to 5 wt.%, preferably 1.5 to 4.5 wt.%, more preferably 1.75 to 3.75% wt.%, preferably 0.75, 1.75,1.25, 2.25, 2.75, 3.25, 3.75, 4.25, 4.5, 4.77 wt.% of a binder, most preferably 1.75 based on the total weight of the immediate release layer a). The filler, the disintegrant, the lubricant, the glidant and the binder are preferably selected from the above-mentioned compounds. The immediate release layer a) may further contain 3 to 6 wt.%, preferably 4 to 5 wt.%, more preferably 4.5 to 4.9 wt.%, such as 4.9 wt.%, of ramipril and can consist of ramipril and the aforementioned excipients in the indicated amounts. Even more preferably, the immediate release layer a) contains or consist of 4.9% of ramipril, 86.35 wt.% of Lactose monohydrate, 5 wt.% of Croscarmellose sodium, 1.75 wt.% Polyvinyl alcohol and 2 wt.% of Stearyl fumarate sodium, based on the total weight of the immediate release layer a)

According to another particularly preferred embodiment, the sustained-release layer b) comprises 25 to 45 wt.%, preferably 30 to 42 wt.%, more preferably 32 to 37 wt.%, of the sustained-release agent, 50 to 70 wt.%, preferably 52 to 69 wt.%, more preferably 57 to 63 wt.% of a filler, 4.0 to 5.0 wt.%, preferably 4.2 to 5.5 wt.%, most preferably 4.3 wt.% of a binder, 0.2 to 0.6 wt.%, preferably 0.3 to 0.5 wt.%, most preferably 0.5 wt.% of a lubricant, and 0.1 to 0.5 wt.%, preferably 0.2 to 0.3 wt.%, most preferably 0.2 wt.% wt.% of a glidant, based on the total weight of the sustained-release layer b).

Preferably, it does not contain a disintegrant. The sustained-release agent, the filler, the lubricant, the glidant and the binder are preferably selected from the above-mentioned compounds. The sustained release layer b) may further contain 0.25 to 2 wt.%, preferably 0.5 to 1.50 wt.%, more preferably 0.75 to 1.25 wt.%, such as 0.75 wt.%, of indapamide and the consist of indapamide and the aforementioned excipients in the indicated amounts. More preferably the sustained-release layer b) contains or consists of 0.75 wt.% of indapamide, 52.25 wt.% of lactose monohydrate, 4,30 wt.% of polyvinylpyrrolidone, 42,00 wt.% of Hypromellose K4M, 0.20 wt.% of colloidal anhydrous silica, and 0.50 wt.% of magnesium stearate based on the total weight of the sustained release layer b) or the sustained-release layer b) contains or consists of 0.75 wt.% of indapamide, 52.25 wt.% of lactose monohydrate, 4,30 wt.% of polyvinylpyrrolidone, 22,00 wt.% of Hypromellose K4M, 20,00 wt.% of Hypromellose E4M 0.20 wt.% of colloidal anhydrous silica, and 0.50 wt.% of magnesium stearate based on the total weight of the sustained release layer b). Even more preferably, the sustained-release layer b) contains or consists of 0.75 wt.% of indapamide, 62.25 wt.% of lactose monohydrate, 4,30 wt.% of polyvinylpyrrolidone, 32,00 wt.% of Hypromellose K4M, 0.20 wt.% of colloidal anhydrous silica, and 0.50 wt.% of magnesium stearate based on the total weight of the sustained release layer b) or the sustained-release layer b) contains or consists of 0.75 wt.% of indapamide, 62.25 wt.% of lactose monohydrate, 4,30 wt.% of polyvinylpyrrolidone, 20,00 wt.% of Hypromellose K4M, 12,00 wt.% of Hypromellose E4M 0.20 wt.% of colloidal anhydrous silica, and 0.50 wt.% of magnesium stearate based on the total weight of the sustained release layer b).

In a preferred variant of the pharmaceutical composition, the IR layer and SR layer form a bilayer tablet and the SR layer contains HPMC or HEC matrix allowing for the sustained release of indapamide. The sustained release of indapamide may be further characterized by the thickness of the HPMC or HEC matrix after swelling, i.e. in the swollen state.

After immersing the dry sustained release layer b) in a 30 to 35 mL of a medium: pH=1.2 (0.1M aqueous HCl solution) for 1 hour and subsequently in a 30 to 35 mL of a medium phosphate buffer pH=4,5 for 1 h, contained in beaker the beaker being placed in a wiggled, heated bath at 37°C, the matrix of the sustained-release layer b) is in a swollen state and has a thickness in the range of 6.5-8.0 mm, preferably 6.7 -7.3 mm.

The thickness measurements are performed on the SR layer, swollen in a medium resembling conditions in vivo. Such a thickness of the swollen SR layer is favourable for obtaining a sustained release of indapamide from the HPMC matrix in order to obtain a desirable time/concentration profile of indapamide. Before swelling, the dry sustained-release layer has a thickness in the range of 3.0 to 5.0 mm, preferably 3.2 - 3.5 mm. The thickness of the sustained-release layer b) in the swollen state is in a range of 6.5-8.0 mm, preferably 6.7 -7.3 mm, and for such a swollen matrix the work parameter is below 38 -37 N*mm.

Preferably the pharmaceutical composition of the present invention has an in vitro dissolution rate dissolution release rate of indapamide of 10 to 30 wt.%, preferably 12 to 19 wt.% of indapamide within 2 h, 40 to 65 wt.% within 8 h, 45 to 65wt.% within 10h and above 85 wt.% within 21 h, based on the total indapamide content as determined by using European Pharmacopoeia edition 8 Apparatus Paddles "2" in 900 mL volume of Phosphate buffer pH 6.8 at 37.0°C±0.5°C and 50 rpm, and dissolution rate of ramipril of 85 wt.%, preferably 90 wt.%, most preferably 97wt.% of ramipril within 15 minutes, based on the total ramipril content as determined by using European Pharmacopoeia edition 8 Apparatus Baskets "1" in 500 mL volume of 0.1 M HCl (pH 1.2) at 37.0°C±0.5°C and 100 rpm.

In another aspect, the invention relates to the method of manufacturing the prolonged-release pharmaceutical composition. The method enables the production of the pharmaceutical composition using conventional tools and well-known pharmaceutical technologies, while significantly simplifying the manufacturing process, and reducing its costs comparing to manufacturing standards known in the art. The pharmaceutical composition, in particular in the form of a bilayer tablet, can be prepared by a method comprising direct compression, dry granulation or wet granulation for making the immediate-release layer and the sustained-release layer b).

Thus, a method of manufacturing the prolonged-release pharmaceutical composition comprises (a) a step of preparing the immediate release layer a); (b) a step of preparing the sustained-release layer b); (c) a step of combining the immediate release layer a) and the sustained-release layer (b) to form a bilayer tablet, in which the sustained-release layer (b) forms a first layer, and the immediate release layer a) the forms a second layer; and, optionally, (d) a step of forming a coating around the first and second layers obtained in step (c). Preferably, a coating is formed, that is, step (d) is preferably carried out.

In step (a) and step (b), the IR layer and SR layer may be independently prepared using a dry or wet technology such as dry or wet granulation.

A dry-granulated component of the IR layer or SR layer can be obtained using direct dry granulation of a powder mixture comprising ramipril or indapamide or a pharmaceutically acceptable salt thereof, suitable excipients as described above and, in case of the SR layer, a sustained-release agent, as described above, in particular at least one of hydroxypropylmethylcellulose (HPMC) or hydroxyethylcellulose (HEC) grades with a viscosity in a range of 2 500 - 5 500 mPas, preferably 2 700 - 5 500, most preferably 2500 - 5 040 mPas, such as 4 000 mPas measured according to EU Pharmacopoeia 7.0 method 2.2.8 in 2wt.% water solution. Preferably, the sustained release agent is a HPMC grade K4M or E4M or HEC grade HHX. Other excipients can be also included, if needed. Suitable excipients, used in pharmacy, can be found in the "Handbook of Pharmaceutical Excipients" edited by R. C. Rowe, P. J. Sheskey, and S. Owen, Pharmaceutical Press, edition VII. The dry granulation method used to obtain the component of the IR layer (step (a)) or the component of the SR layer (step (b)) can be selected from roller compaction and slugging. Methods known to the skilled person and are described, for example, in the "Pharmaceutical Manufacturing Handbook" edited by Shayne Cox Gad, published by John Wiley & Sons, Inc., Hoboken, New Jersey.

A wet-granulated components of the IR layer or components of the SR layer can be obtained using a suitable granulation method known in the art using powder mixtures comprising ramipril or indapamide or a pharmaceutically acceptable salt thereof, suitable excipients as described above and, in case of the SR layer, a sustained-release agent, as described above, in particular at least one of hydroxypropylmethylcellulose (HPMC) or hydroxyethylcellulose (HEC) grades with a viscosity in a range of 2 500 - 5 500 mPas, preferably 2 700 - 5 500 mPas, most preferably 2500 - 5 040 mPas, such as 4000 mPas measured according to EU Pharmacopoeia 7.0 method 2.2.8 in 2wt.% water solution. Other excipients can be also included, if needed. The wet granulation method used to obtain the components of the IR layer (step (a)) or the components of the SR layer (step (b)) can be selected from low shear, high shear, and fluid bed granulation. Preferably, the components of the IR layer and components of the SR layer are obtained by wet granulation (steps (a) and (b)).

Blending operations can be performed in a suitable blender, preferably in a container blender.

According to an embodiment of the method, the components of IR layer a) and/or the SR layer b) are prepared using dry granulation (steps (a) and (b)). In such a variant, the method preferably comprises the following steps: (I) blending ramipril or a pharmaceutically acceptable salt thereof with all excipients except lubricant and, in case of the components of the SR layer, with the at least one sustained-release agent to homogeneity; II) adding a part of the lubricant and subsequent blending; III) dry granulation by roller-compaction or slugging; IV) breaking-down the resulting slugs or sheets using a milling technique to produce granules; V) adding the remaining part of lubricant and subsequent blending.

In the dry methods of granulation, the primary powder blend particles are aggregated under high pressure using one of two main dry granulation processes: either roller-compaction or slugging. In both cases the intermediate products, slugs or sheets, are broken down using a suitable milling technique to produce granular material, which can be additionally sieved to separate the desired size fraction.

According to another embodiment of the method, the IR layer a) and/or the SR layer b) are prepared using wet granulation (steps (a) and (b)) and subsequent compression to the respective layers. In such a variant, the method preferably comprises the following steps: I) mixing of ramipril or a pharmaceutically acceptable salt thereof with at least one filler, at least one disintegrant, optionally further excipients, and, in case of the components of the SR layer, at least one sustained-release agent in a high-shear granulator to homogeneity; II) granulating the mixture of step I) by addition of a binder solution in water or another processing agent, such as an alcohol, e.g. ethanol, or with a mixture thereof; III) drying and unifying the dried granule sizes by sieving or screening; and, optionally, IV) adding an extragranular phase comprising lubricant and/or a mixture of filler and glidant and/or an absorbent and a glidant.

Ramipril and Indapamide may be also subjected to a process of wet granulation (e.g. low shear, high shear or in a fluidized bed), to improve its solubility. Wet granulation facilitates obtaining the desired in vitro and in vivo release profiles. The resulting wet granulate is dried and, for example, screened to obtain a proper distribution of the particles, allowing a durable combination with the other component and additional excipients.

According to a preferred embodiment of the method, the IR layer a) is prepared using wet granulation (step (a)), and the components of SR layer b) is prepared using wet granulation (step (b)).

In step (c) of the method, the resulting components of immediate release layer a) and sustained release layer b) are combined together, and compressed into separate layers in a tablet, optionally followed by forming a coating in step (d), resulting in a single unit dosage form. Preferably, the final fixed dose pharmaceutical composition of ramipril and indapamide is prepared by using a bilayer tableting technology in step (c).

### Examples

### Example 1. Preparation of IR layer, SR layer and pharmaceutical composition

Exemplary IR layers (IR1 to IR8) and SR layers (SR1 to SR/9) according to the present invention are presented in the Tables 1 and 2 below.

The composition of the 10/1.5 mg and 5/1.5 mg Ramipril IR /Indapamide SR strengths are quantitively proportional, i.e. the ratio between the amount of each excipient to the amount of active substance in the particular tablet layer is the same for both strengths.

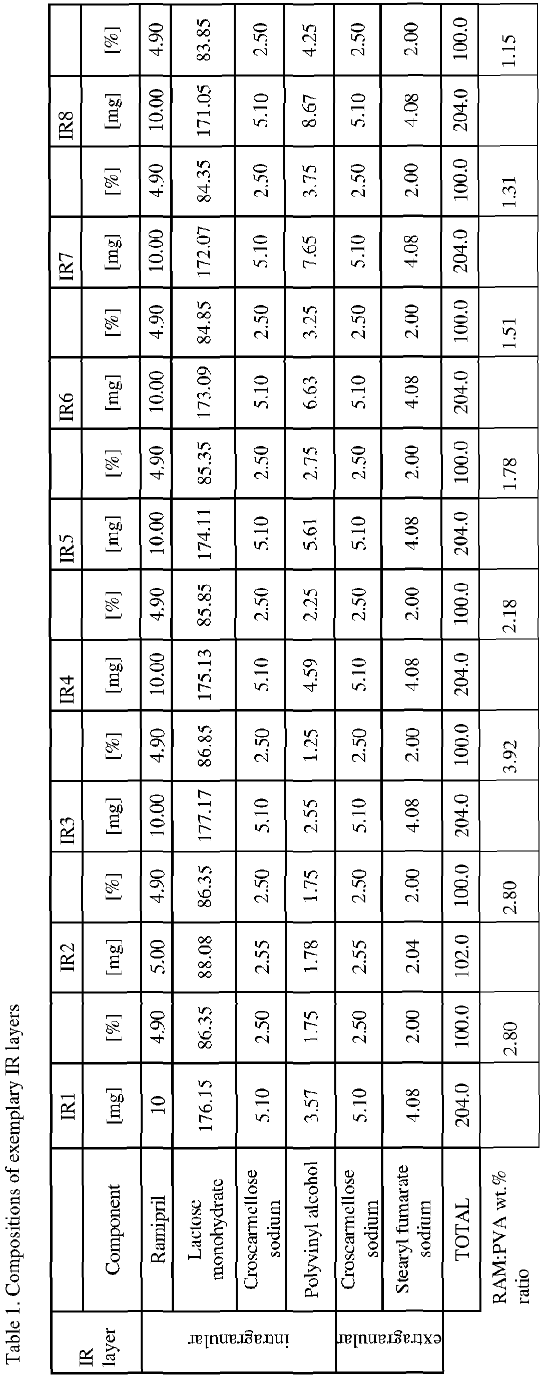

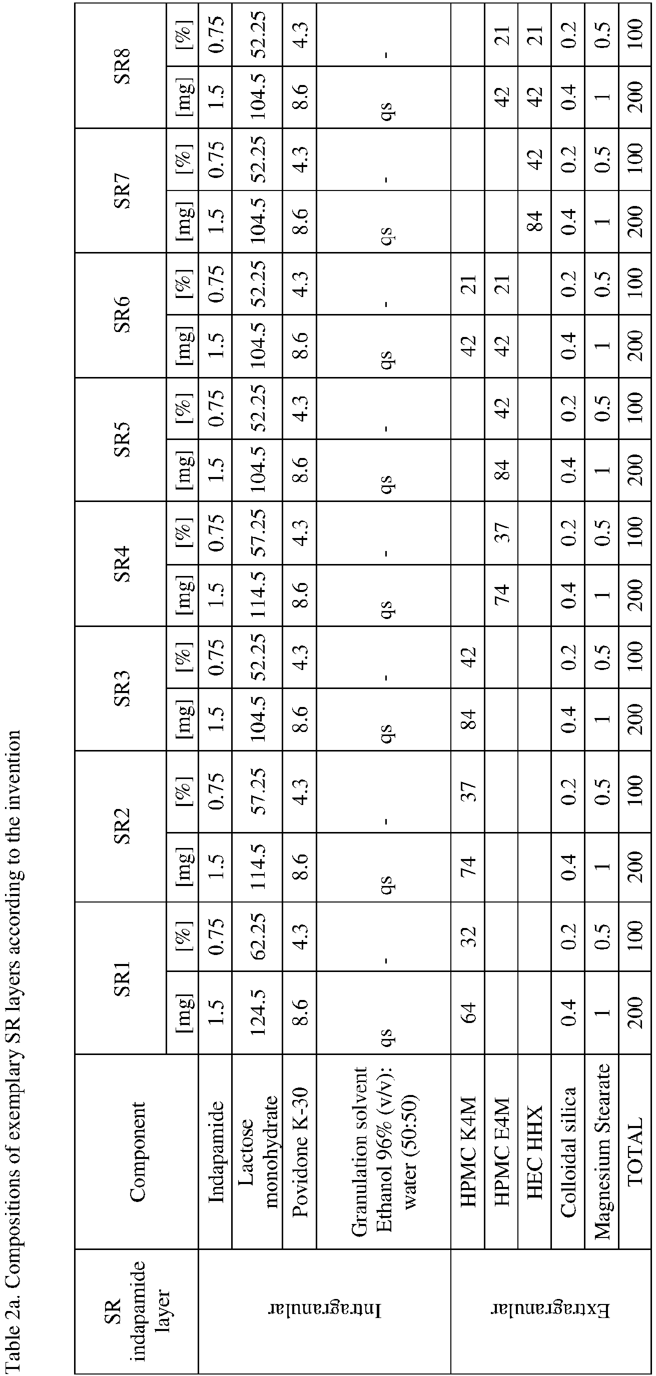

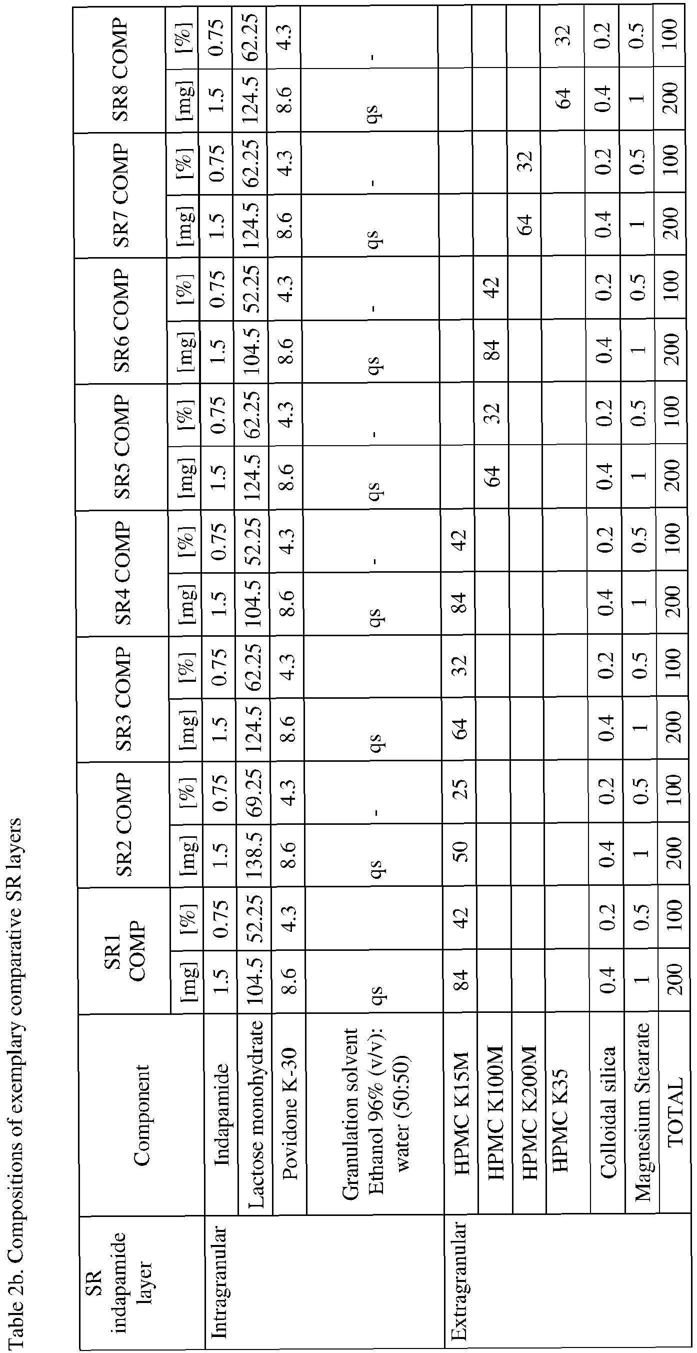

In the following procedures, the amounts of the components were used as specified in Tables 1 and 2. As the coating commercial Opadry II (Opadry II 85F18422) was used in amount 12,12 mg per tablet (3,00 wt.% calculated on the total weight of the bilayer tablet) the tablet cores are coated to a weight gain of about 3% with a tablet coater such as Glatt GC Smart equipped with full perforated coating drum.

### Preparation of granulate of components of the IR layer

The granulate further compressed to IR layers are prepared as follows:
1. Weighing of ramipril, Lactose monohydrate, Croscarmellose sodium, Poly(vinyl alcohol), Purified Water, (i.e. the active substance, filler, disintegrant, binder and processing agent).
2. Mixing ramipril, Lactose monohydrate, 50% of the Croscarmellose sodium in a high shear granulator (i.e. the active substance, filler, disintegrant).
3. Preparation of the binder solution: Poly(vinyl alcohol), purified water (i.e. binder, processing agent)
4. Wet granulation using the mixture from step 2 and the binder solution from step 3 is performed in a High Shear Granulator. Granulation time 1 to 3 min. Appearance of the wet blend was assessed by squeezing the mass.
5. Wet-sizing of the granulate obtained in step 4 in a Conical screen mill, IPC - Screen size 3 to 6 mm.
6. Drying of the seized granulate obtained step 5 in a fluid bed dryer at a temperature of max. 40°C, accepted levels of LOD are in a range of 0.5 to 2 wt.%.
7. Sizing of the granulate obtained in step 6 in a sizing machine, IPC - Screen size 0.8-1.5 mm
8. Weighing of the remaining 50% of Croscarmellose sodium (i.e. the disintegrant).
9. Blending the sized granulate obtained in step 7 with 50% of Croscarmellose sodium In a bin blender. Blending time 15-25 min.
10. Blending the granulate from step 9 with Sodium stearyl fumarate (lubricant) in a bin blender. Blending time 3 to 5 min.

### Preparation of granulate of components of the SR layer

The SR layers are prepared as follows:
1. Weighing of indapamide, lactose monohydrate, Purified Water, Ethanol, Povidone K-30 (i.e. the active substance, filler, binder, lubricant, disintegrant and processing agent).
2. Mixing indapamide, povidone K-30,and lactose monohydrate in a high shear granulator (i.e. indapamide, filler, agent and disintegrant).
3. Preparation of the binder solution: mixing purified water and ethanol (i.e. binder and processing agents)
4. Wet Granulation using the mixture from step 2 and the binder solution from step 3 is performed in a high shear granulator. Granulation time 1 to 3 min. Appearance of the wet blend was assessed by squeezing the mass.
5. Wet-sizing of the granulate obtained in step 4 in a sizing machine, IPC - Screen size 3 to 6 mm.
6. Drying of the seized granulate obtained in step 5 in a fluid bed dryer at a temperature of max. 55°C, accepted levels of LOD are in a range of 0.5 to 1.5%.
7. Sizing of the granulate obtained in step 6 in a sizing machine, IPC - Screen size 0.5-1.5 mm
8. Blending the sized granulate obtained in step 7 with Hypromellose and Colloidal silica (i.e. sustained release agent, glidant)
9. Blending the granulate from step 8 with magnesium stearate (i.e. lubricant) in a bin blender. Blending time 3 to 5 min.

### Preparation of bilayer tablets

The final blend granulates are compressed into bilayer-tablets with the aid of rotary press machine. SR layer is the first layer in the bilayer tablet. During the compression process, the following requirements of the tablets should be satisfied (Table 3).

**Table 3. Requirements during compression process**

| Parameters of tablet cores | Requirements |
|---|---|
| Appearance | round, biconvex, white to white of, smooth tablet cores |
| Average mass of sustained release layer | 200 mg (190 - 210 mg) |
| Average mass of immediate release layer | 102 or 204 mg (97 - 107 or 194- 214 mg) |
| Average mass of tablet cores | 302 or 404 mg (287- 317 or 384- 424 mg) |
| Hardness of tablet cores | 60 - 150 N |
| Friability | NMT 1.0% |

For the pharmaceutical composition IR1+SR1 and IR2+SR1the above processes were used to prepare a batches of bilayer tablets using 51 kg of the granulate of the components of sustained-release layer and 39 kg of the granulate of components of immediate release layer.

### Coated of the bilayer tablets

Specified amount of film-coating system is added into a container with purified water while stirring and mixed . The obtained tablet cores are film-coated and Ramipril/Indapamide bilayer film coated tablets are obtained.

### Example 2. Dissolution studies

The pharmaceutical compositions according to the present invention IR1/SR4, IR2/SR1, IR3/SR4, IR1/SR1, IR2/SR8, IR2/SR6 and the marketed referent formulations of ramipril Tritace 10mg and Indapamide [Tertensif SR 1.5 mg], as well as comparative examples IR2/SR3COM and IR2SR2COM were subjected to dissolution tests carried out in accordance with the test methods described in Table 4, below

**Table. 4 Dissolution testing parameters**

| Parameter | Dissolution testing conditions for Indapamide | Dissolution testing conditions for Ramipril |
|---|---|---|
| Medium: | Phosphate buffer pH 6.8 | 0.1 M HCl (pH 1.2) |
| Volume [ml]: | 900 | 500 |
| Apparatus type: | Apparatus type: 2 (paddles with sinkers, according to European Pharmacopoeia 8^{th} edition) | Apparatus type: 1 (baskets, according to European Pharmacopoeia 8^{th} edition) |
| Temperature | 37°C +- 0.5°C | 37°C +- 0.5°C |
| RPM: | 50 | 100 |
| Time points [min]: | 60, 120, 180, 240, 360, 480, 600, 720, 900, 1080, 1260, 1440 | 10, 15, 20, infinity test |

The quantities of drug substance released from the tested tablets are determined by HPLC. The mean value calculated from 6 tablets are indicated in Tables below. Requirements are presents in Table 5.

**Table 5.**

| | REQUIREMENTS | IR1/SR4 | IR2/SR1 | referent Tertensif SR 1.5 mg or Tritace 10mg |
|---|---|---|---|---|
| Dissolution of ramipril | After 15 minutes: | After 15 minutes | After 15 minutes | After 15 minutes |
| | for each tablet ≥ 85% | Average value 97.1 wt.% min. value 94.9 wt.% | Average value 95.3 wt.% | Average value 90 wt.% |
| | (S₁= Q + 5; Q = 80%) | max. value 99.4 wt.% | min. value 86.2 wt.% | min. value 87 wt.% |
| | | | max. value 98.6% | max. value 92 wt.% |
| | The additional test is acceptable | | | |
| Dissolution of indapamide | Each tablet | - 2 hour | --- | - 2 hour |
| | - 2 hour: 10 wt.% < U < 30 wt.% | mean = 16.8 wt.% min = 15.0 wt.% | | Mean = 9.2% |
| | | | | Min = 7.6% |
| | - 8 hours: 45 wt.% < U < 65 wt.% | max = 18.6 wt.% | | Max =10.2% |
| | | - 8 hours | | - 8 hours |
| | - 21 hours: >= 85 wt.% (Q=80 wt.%) | Mean = 56.5 wt.% | | Mean =52.1 |
| | | Min = 51.6 wt.% | | Min = 49.8% |
| | | Max = 61.1% - 21 hours | | Max = 54.3% - 21 hours |
| | | Mean = 93.4 wt.% | | Mean = 91.5% |
| | | Min = 88.8 wt.% | | Min = 82.4% |
| | | Max = 94.1 | | Max =95.5% |
| | Each tablet | --- | - 2 hour | - 2 hour |
| | - 2 hour: 10 wt.% < U < 30 wt.% | | mean = 13.8 wt.% min = 12.3% | Mean = 9.2% |
| | | | | Min = 7.6% |
| | - 10 hours: 45 wt.% < U < 65 wt.% | | max = 15.1% | Max =10.2% |
| | | | - 10 hours | - 10 hours |
| | | | Mean = 57.3% | Mean =64.1% |
| | - 24 hours: >= 80 wt.% (Q=75 wt.%) | | Min = 51.2 wt.% | Min = 58.8% |
| | | | Max = 61.9% - 24 hours | Max = 69.5% - 24 hours |
| | | | Mean = 91.0% | Mean =95.1% |
| | | | Min = 83.8 wt.% | Min = 86.4% |
| | | | Max = 96.1% | Max =100.1% |

**Table 6. Dissolution testing for tablets RAM/IND tablets 10 mg / 1.5 mg**

| Dissolution Data - 0.1 M HCl | | | Dissolution Data - phosphate buffer pH 6.8 | | | Dissolution Data - 0.1 M HCl | | | Dissolution Data - phosphate buffer pH 6.8 | | |
|---|---|---|---|---|---|---|---|---|---|---|---|
| RAM/IND tablets 10 mg / 1.5 mg - RAM IR3 | | | RAM/IND tablets 10 mg / 1.5 mg - IND SR4, f2 factor- 62.3 | | | Ramipril reference tablets, Tritace 10 mg | | | Indapamide reference tablets, Tertensif SR 1.5 mg | | |
| Time point [min] | Mean [%] | RSD [%] | Time point [min] | Mean [%] | RSD [%] | Time point [min] | Mean [%] | RSD [%] | Time point [min] | Mean [%] | RSD [%] |
| 0 | 0 | 0 | 0 | 0 | 0 | 0 | 0 | 0 | 0 | 0 | 0 |
| 10 | 94.0 | 4.9 | 60 | 8.4 | 9.0 | 10 | 99.3 | 2.2 | 60 | 3.9 | 8.7 |
| 15 | 97.1 | 1.7 | 120 | 16.8 | 8.4 | 15 | 99.4 | 2.3 | 120 | 9.2 | 8.4 |
| 20 | 97.6 | 1.4 | 180 | 24.6 | 7.9 | 20 | 99. 5 | 2.3 | 180 | 16.0 | 8.3 |
| inf | 98.2 | 1.2 | 240 | 32.0 | 7.5 | inf | 99.4 | 2.3 | 240 | 23.3 | 6.9 |
| | | | 360 | 45.2 | 7.3 | | | | 360 | 38.0 | 4.4 |
| | | | 480 | 56.5 | 7.1 | | | | 480 | 52.1 | 2.8 |
| | | | 600 | 65.9 | 6.7 | | | | 600 | 64.1 | 5.2 |
| | | | 720 | 73.5 | 6.3 | | | | 720 | 72.3 | 6.5 |
| | | | 900 | 82.3 | 5.6 | | | | 900 | 80.4 | 6.3 |
| | | | 1080 | 88.8 | 4.9 | | | | 1080 | 86.6 | 5.3 |
| | | | 1260 | 93.4 | 4.3 | | | | 1260 | 91.5 | 4.5 |
| | | | 1440 | 96.7 | 3.9 | | | | 1440 | 95.1 | 3.9 |

**Table 7. Dissolution testing for tablets RAM/IND tablets 10 mg / 1.5 mg**

| Dissolution Data - 0.1 M HCl | | | Dissolution Data - phosphate buffer pH 6.8 | | | Dissolution Data - 0.1 M HCl | | | Dissolution Data - phosphate buffer pH 6.8 | | |
|---|---|---|---|---|---|---|---|---|---|---|---|
| RAM/IND tablets 10 mg / 1.5 mg - RAM IR1 | | | RAM/IND tablets 10 mg / 1.5 mg - IND SR1 f2 factor=64.6 | | | Ramipril reference tablets, Tritace 10 mg | | | Indapamid reference tablets, Tertensif SR 1.5 mg | | |
| Time point [min] | Mean [%] | RSD [%] | Time point [min] | Mean [%] | RSD [%] | Time point [min] | Mean [%] | RSD [%] | Time point [min] | Mean [%] | RSD [%] |
| 0 | 0 | 0 | 0 | 0 | 0 | 0 | 0 | 0 | 0 | 0 | 0 |
| 10 | 95.3 | 3.4 | 60 | 6.7 | 7.3 | 10 | 99.3 | 2.2 | 60 | 3.9 | 8.7 |
| 15 | 95.3 | 4.7 | 120 | 13.8 | 6.83 | 15 | 99.4 | 2.3 | 120 | 9.2 | 8.4 |
| 20 | 97.7 | 1.2 | 180 | 20.5 | 6.9 | 20 | 99.5 | 2.3 | 180 | 16.0 | 8.3 |
| inf | 98.2 | 1.4 | 240 | 26.9 | 6.9 | inf | 99.4 | 2.3 | 240 | 23.3 | 6.9 |
| | | | 360 | 38.3 | 6.9 | | | | 360 | 38.0 | 4.4 |
| | | | 480 | 48.4 | 6.8 | | | | 480 | 52.1 | 2.8 |
| | | | 600 | 57.3 | 6.6 | | | | 600 | 64.1 | 5.2 |
| | | | 720 | 64.9 | 6.3 | | | | 720 | 72.3 | 6.5 |
| | | | 900 | 74.0 | 6.1 | | | | 900 | 80.4 | 6.3 |
| | | | 1080 | 81.1 | 5.8 | | | | 1080 | 86.6 | 5.3 |
| | | | 1260 | 86.61 | 5.5 | | | | 1260 | 91.5 | 4.5 |
| | | | 1440 | 91.0 | 5.2 | | | | 1440 | 95.1 | 3.9 |

**Table 8. Dissolution testing for tablets RAM/IND tablets 5 mg / 1.5 mg**

| Dissolution Data - 0.1 M HCl f2 = 56.1 | | | Dissolution Data - phosphate buffer pH 6.8 | | | Dissolution Data - 0.1 M HCl f2 = 62.0 | | | Dissolution Data - phosphate buffer pH 6.8 | | |
|---|---|---|---|---|---|---|---|---|---|---|---|
| RAM/IND tablets 5 mg / 1.5 mg - RAM IR2 | | | RAM/IND tablets 5 mg / 1.5 mg - IND SR8 | | | RAM/IND tablets 5 mg / 1.5 mg - RAM IR2 | | | RAM/IND tablets 5 mg / 1.5 mg - IND SR6 | | |
| Time point [min] | Mean [%] | RSD [%] | Time point [min] | Mean [%] | RSD [%] | Time point [min] | Mean [%] | RSD [%] | Time point [min] | Mean [%] | RSD [%] |
| 0 | 0 | 0 | 0 | 0 | 0 | 0 | 0 | 0 | 0 | 0 | 0 |
| 10 | 91.2 | 3.6 | 60 | 6.5 | 4.1 | 10 | 90.6 | 2.2 | 60 | 9.5 | 7.0 |
| 15 | 92.8 | 2.5 | 120 | 13.0 | 4.3 | 15 | 92.5 | 1.6 | 120 | 17.6 | 7.3 |
| 20 | 93.6 | 2.3 | 180 | 19.1 | 4.7 | 20 | 93.4 | 1.2 | 180 | 25.3 | 8.1 |
| inf | 94.7 | 2.1 | 240 | 25.0 | 5.0 | inf | 94.5 | 1.0 | 240 | 32.3 | 8.3 |
| | | | 360 | 35.8 | 5.5 | | | | 360 | 44.6 | 7.9 |
| | | | 480 | 45.3 | 5.4 | | | | 480 | 55.1 | 7.8 |
| | | | 600 | 53.5 | 5.3 | | | | 600 | 63.9 | 7.5 |
| | | | 720 | 60.6 | 5.2 | | | | 720 | 71.4 | 7.2 |
| | | | 900 | 69.4 | 5.1 | | | | 900 | 80.4 | 6.7 |
| | | | 1080 | 76.4 | 5.1 | | | | 1080 | 87.4 | 6.3 |
| | | | 1260 | 82.3 | 5.0 | | | | 1260 | 92.8 | 5.7 |
| | | | 1440 | 87.3 | 4.9 | | | | 1440 | 96.9 | 5.0 |

**Table 9. Dissolution testing for tablets RAM/IND tablets 5 mg / 1.5 mg with comparative SR layers**

| Dissolution Data - | | | Dissolution Data - | | | Dissolution Data - 0.1 | | | Dissolution Data - | | |
|---|---|---|---|---|---|---|---|---|---|---|---|
| RAM/IND tablets 5 mg / 1.5 mg - RAM IR2 | | | RAM/IND tablets 5 mg / 1.5 mg - IND SR2COMP | | | RAM/IND tablets 5 mg / 1.5 mg - RAM IR2 | | | RAM/IND tablets 5 mg / 1.5 mg - IND - IND SR3COMP | | |
| | | | F2 factor vs referent = 47.2 - not comparable to the referent | | | | | | F2 factor vs referent = 48.0 - not comparable to the referent | | |
| Time | Mean | RSD | Time | Mean | RSD | Time | Mean | RSD | Time | Mean | RSD |
| 0 | 0 | 0 | 0 | 0 | 0 | 0 | 0 | 0 | 0 | 0 | 0 |
| 10 | 90.2 | 3.2 | 60 | 5.6 | 6.5 | 10 | 90.5 | 2.0 | 60 | 5.8 | 7.5 |
| 15 | 92.5 | 2.4 | 120 | 11.2 | 6.5 | 15 | 91.5 | 1.7 | 120 | 11.2 | 5.0 |
| 20 | 92.6 | 2.3 | 180 | 16.8 | 6.8 | 20 | 93.6 | 1.5 | 180 | 16.5 | 4.6 |
| inf | 95.7 | 2.0 | 240 | 22.1 | 6.6 | inf | 95.5 | 1.1 | 240 | 21.7 | 4.8 |
| | | | 360 | 31.9 | 6.3 | | | | 360 | 31.4 | 4.8 |
| | | | 480 | 40.5 | 6.3 | | | | 480 | 40.4 | 4.6 |
| | | | 600 | 48.2 | 6.4 | | | | 600 | 48.2 | 4.2 |
| | | | 720 | 54.9 | 6.3 | | | | 720 | 55.5 | 4.6 |
| | | | 900 | 63.4 | 6.2 | | | | 900 | 64.5 | 4.5 |
| | | | 1080 | 70.5 | 6.1 | | | | 1080 | 71.9 | 4.7 |
| | | | 1260 | 76.4 | 6.1 | | | | 1260 | 78.0 | 4.9 |
| | | | 1440 | 81.4 | 6.0 | | | | 1440 | 83.1 | 5.1 |

The amount of ramipril dissolved from the tablets according to the present invention and the reference drug product Tritace 10mg was above 85 wt.% within 15 minutes, thus the dissolution profiles of API from both products are assumed similar without further mathematical evaluation according to *"Guideline on the Investigation of Bioequivalence"* (CPMP/EWP/QWP/1401/98 Rev. 1/Corr**).

The Indapamide dissolution profiles from the tested tablets according to the present invention and reference drug products (Tertensif SR 1.5 mg) were assessed in a buffer at pH 6.8 on the basis of the calculated similarity factor f2. The obtained value of f2 factors > 50 (such 62.3 or 64.6) confirm that the dissolution profiles are similar.

The calculated similarity factor f2 values for the Indapamide dissolution profiles for comparative SRCOMP compositions were <50, meaning that these compositions are not suitable to be used in treatments where Indapamide is required.

### Example 3. Swelling test - analysis of the SR matrix texture

In Example 3, the swelling properties of the SR layer having a HPMC or HEC polymer matrix and the dissolution of indapamide from the SR layer were investigated. For the swelling test, the samples were prepared as follows. The test time and conditions are adjusted as to achieve complete dissolution of the IR layer and mimic the swelling characteristics of the SR layer after 2 h mean retention time of the tested tablet in the stomach.

Sample preparation: Tablets (n=5) are placed in beaker filled with medium: pH=1.2 (0.1M aqueous HCl solution). The beaker is placed in wiggled, heated bath at 37°C temperature. After 1 hour incubation medium is changed to phosphate buffer pH=4,5. The tablets are incubated for the next 1 hours. The test time and conditions are adjusted to achieve complete dissolution of the IR layer and swelling characteristic of the MR layer after 2h in fasted condition.

The swollen samples were tested using a common texture analyser. Before testing, the probe of the texture analyser was calibrated against the testing platform (i.e. a blank slide).

After the IR layer of the tablet was completely dissolved (completion after 2 h incubation), the swollen SR layer (i.e. the SR layer is the swollen state) of the tablets was transferred from the beaker on slides and singly tested.

For the data analysis, the values of interest for a sample were obtained by a macro included in the software of the TA.XTplus Texture Analyser. The macro quantifies the measured gel thickness and total work of probe penetration into the entire swollen matrix. The average values of 5 tablets were calculated. The results are summarized in Table 5 indicating the thickness of the SR layer before swelling, the thickness range of the individual results after swelling, the average thickness after swelling. The following settings were used:
Instrument/software: TA.XTplus Texture Analyser (Stable Micro Systems Ltd)/Exponent
Accessory: Load cell - 5 kg; Cylinder probe
TA settings: Mode: Measure Distance in Compression; Pre-Test Speed: 1.0 mm/s'; Test speed: 0.1 mm/s Post-Test Speed: 5.0 mm/s; Target Mode: Force.

**Table 10. Thickness of the initial and swollen SR layer and work parameter of the SR layer after incubation for 1h in pH=1.2 and 1h in phosphate buffer pH=4,5 at 37°C 1 hour.**

| | Work [N*mm] | Thickness | Thickness | Thicknes |
|---|---|---|---|---|
| IR1/SR1 | 27.25 | 7.1 - 7.6 | 7.3 | 3.2 |
| IR2/SR4 | 33.97 | 6.5 - 7.2 | 6.8 | 3.2 |
| IR1/SR2 | 37.02 | 6.7 - 6.9 | 6.7 | 3.2 |
| IR2/SR8 | 32.58 | 7.2 - 7.5 | 7.3 | 3.2 |
| IR2/SR1 | 30.12 | 6.6 - 7.1 | 6.9 | 3.2 |
| IR5/SR1 | 35.04 | 6.6 - 7.4 | 7.0 | 3.2 |
| IR3/SR4 | 27.74 | 7.4 - 8.2 | 7.9 | 3.2 |
| IR2/SR3COM | 40.27 | 5.2 - 5.8 | 5.5 | 3.2 |
| IR2/SR2COM | 44.20 | 5.5 - 5.9 | 5.7 | 3.2 |
| IR1/SR1COM | 41.60 | 5.1 - 5.5 | 5.3 | 3.2 |
| IR1/SR7COM | 42.20 | 5.0 - 5.2 | 5.1 | 3.2 |

The dissolution amount of indapamide from the SR layer has been determined according to the dissolution test in Example 2. The mean values for several tablets are indicated in section Dissolution.

It was found that the thickness of the swollen SR layer correlated with the dissolution profile of tablets composed of IR layer and SR layer.

The 2h time point of dissolution has been chosen as representative for the correlation and referent for the prediction of the required full dissolution profile. The dissolution values at the representative 2h time point showed a release of indapamide on the level of about 13 to 20 wt.% (a range of 10 to 30 wt.% is acceptable for a prolonged-release composition) for the bilayer tablets composed of IR layer and SR layer. It was found that the results are consistent with the expected levels of released indapamide over time to provide appropriate values in vivo. i.e. levels achieved for the referent Tertensif 1.5 mg SR (Indapamide).

SR layers with indapamide dissolution profile similar to the referent had thickness > 6.7 mm (in a range of 6.5-8.0 mm, preferably 6.7 -7.3 mm). The SR-layers for which work value has been measured as below 38-37 N*mm
Bilayer tablets comprising in the SR layer HPMC with viscosities outside the range 2 500 - 5 500 mPas (Comparative examples, SRCOMP), were characterized with thickness after swelling < 6.7 mm and these SR layers didn't provide required dissolution profiles of indapamide. Therefore the use of HPMC grades with viscosities outside the range of 2 500 - 5 500 mPas in SR indapamide layer will not result in bioequivalent, i.e. useful in medicine, indapamide-comprising composition.

### Example 4. Stability studies

Medicines must comply with the requirements of the chemical purity immediately after preparation (comply with the limits indicated in the release specification of the drug product), and within the period set by the relevant guidelines in time and storage conditions (comply with the limits indicated in the shelf-life specification of the drug product).

The requirements for related substances were established according to guidelines CPCP/ICH/367/96 (ICH Q6A), and CPMP/ICH/2738/99 (ICH Q3B (R2)). The in-house chromatographic method for related substances is selective for degradation products for Ramipril and Indapamide according to Pharmacopoeia. Impurities of ramipril are specified in Pharmacopoeia No 04/2016:1368. Impurities of Indapamid are specified in Pharmacopoeia No 01/2017:1108.

Stability studies with tablets of the invention according to compositions IR1/SR1 and IR2/SR1, packed in PA-aluminium-PVC (laminate) and aluminium foil blister and carton box have been carried out at accelerated conditions (25C/60%RH and 40C/75% RH), the dissolution profiles of the active substances remained substantially unchanged and no changes were observed in the aged formulations.

The results are presented in Tables below:

**Table 11. Stability under long term conditions (25°C, 60% RH) - example IR1/SR1**

| Parameter | RAM/IND bilayer tablet 10 mg / 1.5 mg | | | | | |
|---|---|---|---|---|---|---|
| | Requirements¹ | Start | 1 M | 6M | 12M | 18M |
| **Chromatographic purity** | | | | | | |
| - impurity D RAM | not more than 5.0 wt.% | 0.16 | 0.30 | 0.32 | 0.34 | 0.35 |
| - impurity E RAM | not more than 0.5 wt.% | <0.1% | <0.1% | <0.1% | <0.1% | <0.1% |
| - max single unspecified impurity RAM | not more than 0.2 wt.% | <0.1% | <0.1% | <0.1% | <0.1% | <0.1% |
| - impurity B IND | not more than 1.0 wt.% | <0.1% | <0.1% | <0.1% | <0.1% | <0.1% |
| - max single unspecified impurity IND | not more than 0.5 wt.% | <0.1% | <0.1% | <0.1% | <0.1% | <0.1% |
| - total impurities | not more than 6.5 wt.% | 0.16% | 0.30% | 0.32% | 0.34% | 0.35% |

| | | | | | | |
|---|---|---|---|---|---|---|
| ¹The requirements for the parameters tested during the stability study were established according to guidelines CPMP/ICH/367/96 (ICH Q6A), and CPMP/ICH/2738/99 (ICH Q3B (R2)). | | | | | | |

Content of the active substances Ramipril and Indapamide was stable, <2% of change was observed during the whole period of the test.

**Table 12. Stability under long term conditions (25°C, 60% RH) - example IR2/SR1**

| Parameter | RAM/IND bilayer tablet 5 mg / 1.5 mg | | | | | |
|---|---|---|---|---|---|---|
| | Requirements¹ | Start | 1M | 6M | 12M | 18M |
| **Chromatographic purity** | | | | | | |
| - impurity D RAM | not more than 5.0 wt.% | 0.16 | 0.29 | 0.30 | 0.31 | 0.33 |
| - impurity E RAM | not more than 0.5 wt.% | <0.1% | <0.1% | <0.1% | <0.1% | <0.1% |
| - max single unspecified impurity RAM | not more than 0.2 wt.% | <0.1% | <0.1% | <0.1% | <0.1% | <0.1% |
| - impurity BIND | not more than 1.0 wt.% | <0.1% | <0.1% | <0.1% | <0.1% | <0.1% |
| - max single unspecified impurity IND | not more than 0.5 wt.% | <0.1% | <0.1% | <0.1% | <0.1% | <0.1% |
| - total impurities | not more than 6.5 wt.% | 0.16% | 0.29% | 0.30% | 0.31% | 0.33% |

| | | | | | | |
|---|---|---|---|---|---|---|
| ¹The requirements for the parameters tested during the stability study were established according to guidelines CPMP/ICH/367/96 (ICH Q6A), and CPMP/ICH/2738/99 (ICH Q3B (R2)). | | | | | | |

Content of the active substances Ramipril and Indapamide was stable, <2% of change was observed during the whole period of the test.

**Table 13. Stability under enhanced conditions (40°C, 75% RH) - example IR1/SR1**

| Parameter | RAM/IND bilayer tablet 10 mg / 1.5 mg | | | | |
|---|---|---|---|---|---|
| | Requirements¹ | Start | 1M | 3M | 6M |
| **Chromatographic purity** | | | | | |
| - impurity D RAM | not more than 5.0 wt.% | 0.16 | 1.58 | 1.76 | 2.08 |
| - impurity E RAM | not more than 0.5 wt.% | <0.1% | <0.1% | <0.1% | <0.1% |
| - max single unspecified impurity RAM | not more than 0.2 wt.% | <0.1% | <0.1% | <0.1% | <0.1% |
| - impurity B IND | not more than 1.0 wt.% | <0.1% | <0.1% | <0.1% | <0.1% |
| - max single unspecified impurity IND | not more than 0.5 wt.% | <0.1% | <0.1% | <0.1% | <0.1% |
| - total impurities | not more than 6.5 wt.% | 0.16% | 1.58% | 1.76% | 2.08% |

| | | | | | |
|---|---|---|---|---|---|
| ¹The requirements for the parameters tested during the stability study were established according to guidelines CPMP/ICH/367/96 (ICH Q6A), and CPMP/ICH/2738/99 (ICH Q3B (R2)). | | | | | |

Content of the active substances Ramipril and Indapamide was stable, <2% of change was observed during the whole period of the test.

**Table 14. Stability under enhanced conditions (40°C, 75% RH) - example IR2/SR1**

| Parameter | RAM/IND bilayer tablet 5 mg / 1.5 mg | | | | |
|---|---|---|---|---|---|
| | Requirements¹ | Start | 1M | 2M | 6M |
| **Chromatographic purity** | | | | | |
| - impurity D RAM | not more than 5.0 wt.% | 0.16 | 1.51 | 1.89 | 2.17 |
| - impurity E RAM | not more than 0.5 wt.% | <0.1% | <0.1% | <0.1% | <0.1% |
| - max single unspecified impurity RAM | not more than 0.2 wt.% | <0.1% | <0.1% | <0.1% | <0.1% |
| - impurity B IND | not more than 1.0 wt.% | <0.1% | <0.1% | <0.1% | <0.1% |
| - max single unspecified impurity IND | not more than 0.5 wt.% | <0.1% | <0.1% | <0.1% | <0.1% |
| - total impurities | not more than 6.5 wt.% | 0.16% | 1.51% | 1.89% | 2.17% |

| | | | | | |
|---|---|---|---|---|---|
| ¹The requirements for the parameters tested during the stability study were established according to guidelines CPMP/ICH/367/96 (ICH Q6A), and CPMP/ICH/2738/99 (ICH Q3B (R2)). | | | | | |

Content of the active substances Ramipril and Indapamide was stable, <2% of change was observed during the whole period of the test.

## Claims

1. A pharmaceutical composition for oral administration in the form of a tablet, comprising in a single dosage form:
a) an immediate release layer, comprising ramipril and polyvinyl alcohol in a wt.% ratio in a range of 1.1 to 4.0 and one or more pharmaceutically acceptable excipients; and
b) a sustained-release layer, comprising indapamide, a sustained release agent being hydroxypropylmethylcellulose or hydroxyethylcellulose with a viscosity in a range of 2 500 - 5 500 mPas, measured according to EU Pharmacopoeia 7.0 method 2.2.8 in 2% water solution, and one or more pharmaceutically acceptable excipients.

2. The pharmaceutical composition according to claim 1, wherein in the immediate release layer a) the wt.% ratio of ramipril to polyvinyl alcohol is in a range of 1.15 to 3.9, more preferably 1.7 to 2.85, such as 2.8.

3. The pharmaceutical composition according to claim 1 or 2, wherein the hydroxypropylmethylcellulose or hydroxyethylcellulose has a viscosity in a range of 2 700 - 5 500 mPas, most preferably 2500 - 5 040 mPas, such as 4 000 mPas.

4. The pharmaceutical composition according to any one of claims 1 to 3, wherein the immediate release layer a) comprises 10, 5 or 2.5 mg of ramipril calculated on a ramipril free acid, and the sustained-release layer b) comprises 1.5 mg of indapamid, calculated on indapamide free amide; preferably, the immediate release layer a) comprises 10 or 5 mg of ramipril, and the sustained-release layer b) comprises 1.5 mg of indapamid.

5. The pharmaceutical composition according to any one of claims 1 to 4, wherein the sustained-release layer b) comprises 25 to 45 wt.% of the sustained-release agent, preferably 30 to 42 wt.% and more preferably 32 to 37 wt.% such as 32 wt.% of the sustained-release agent, based on the total weight of the sustained-release layer b).

6. The pharmaceutical composition according to any one of claims 1 to claim 5, wherein the HPMC is at least one selected from the group consisting of hydroxypropylmethylcellulose (HMPC) of grades K4M or E4M and the HEC is Hydroxyethylcellulose of grade HHX or any combination thereof.

7. The pharmaceutical composition according to any one of claims 1 to 6, wherein the immediate release layer a) comprises, as the at least one excipient, at least one of a filler, a disintegrant, a binder and a lubricant and any combination thereof, preferably, the immediate-release layer b) comprises 80-90 wt.% of a filler, 0.5-3.0 wt.% of a lubricant and 0.75 to 5 wt.% of a binder, based on the total weight of the immediate release layer a).

8. The pharmaceutical composition according to any one of claims 1 to 7, wherein the sustained-release layer b) comprises, as the at least one excipient, at least one of a filler, a sustained release agent, a binder, a lubricant, a glidant and any combination thereof, preferably, the sustained-release layer b) comprises 25 to 45 wt.% of the sustained-release agent, 50 to 70 wt.% of a filler, 4.0 to 5.0 wt.% of a binder, 0.2 to 0.6 wt.% of a lubricant, and 0.1 to 0.5 wt.%, of a glidant, based on the total weight of the sustained-release layer b).

9. The pharmaceutical composition according to any one of claims 1 to 8, wherein the pharmaceutical composition has an in vitro dissolution rate of indapamide is in a range of 10 to 30 wt.%, preferably 12 to 19 wt.% of indapamide within 2 h, 40 to 65 wt.% within 8 h, 45 to 65% within 10h and above 85 wt.% within 21 h, based on the total indapamide content as determined by using European Pharmacopoeia edition 8 Apparatus Paddles "2" in 900 mL volume of Phosphate buffer pH 6.8 at 37.0°C±0.5°C and 50 rpm, and dissolution rate of ramipril of 85 wt.%, preferably 90 wt.%, most preferably 97wt.% of ramipril within 15 minutes, based on the total ramipril content as determined by using European Pharmacopoeia edition 8 Apparatus Baskets "1" in 500 mL volume of 0.1 M HCl (pH 1.2) at 37.0°C±0.5°C and 100 rpm.

10. The pharmaceutical composition according to any one of claims 1 to 9, comprising in the sustained release layer b) 1.5 mg of indapamide, 124.5 mg of lactose monohydrate, 8.6 mg of povidone K-30, 64 mg of Hypromellose K4M, 0.4 mg of Silica, Colloidal Anhydrous, 1 mg of Magnesium Stearate and in the immediate release layer a)10 mg of Ramipril, 176.15 mg of Lactose monohydrate, 10.2 mg of Croscarmellose sodium, 3.57 mg of Polyvinyl alcohol, and 4.08 mg of Stearyl fumarate sodium;

11. The pharmaceutical composition according to any one of claims 1 to 9, comprising in the sustained release layer b) 1.5 mg of indapamide, 124.5 mg of lactose monohydrate, 8.6 mg of povidone K-30, 64 mg of Hypromellose K4M, 0.4 mg of Silica, Colloidal Anhydrous, 1 mg of Magnesium Stearate and in the immediate release layer a) 5 mg of Ramipril, 88.08 mg of Lactose monohydrate, 5.1 mg of Croscarmellose sodium, 1.78 mg of Polyvinyl alcohol, and 2.04 mg of Stearyl fumarate sodium.

## Patentansprüche

1. Pharmazeutische Zusammensetzung zur oralen Verabreichung in Form einer Tablette, umfassend in einer Einzeldosisform:
a) eine Schicht zur sofortigen Freisetzung, umfassend Ramipril und Polyvinylalkohol in einem Gewichtsprozentverhältnis im Bereich von 1,1 bis 4,0 und einen oder mehrere pharmazeutisch annehmbare Hilfsstoffe; und
b) eine Schicht zur Langzeitfreisetzung, umfassend Indapamid, wobei eines der Mittel zur Langzeitfreisetzung Hydroxypropylmethylcellulose oder Hydroxyethylcellulose mit einer Viskosität im Bereich von 2.500 - 5.500 mPas ist, gemessen gemäß EU-Pharmakopöe 7.0 Methode 2.2.8 in einer 2%igen Wasserlösung, und einen oder mehrere pharmazeutisch annehmbare Hilfsstoffe.

2. Pharmazeutische Zusammensetzung nach Anspruch 1, wobei in der Schicht zur sofortigen Freisetzung a) das Gewichtsprozentverhältnis von Ramipril zu Polyvinylalkohol in einem Bereich von 1,15 bis 3,9, vorzugsweise 1,7 bis 2,85 liegt, beispielsweise 2,8.

3. Pharmazeutische Zusammensetzung nach Anspruch 1 oder 2, wobei die Hydroxypropylmethylcellulose oder Hydroxyethylcellulose eine Viskosität im Bereich von 2.700 bis 5.500 mPas, besonders bevorzugt 2.500 bis 5.040 mPas aufweist, beispielsweise 4.000 mPas.

4. Pharmazeutische Zusammensetzung nach einem der Ansprüche 1 bis 3, wobei die Schicht zur sofortigen Freisetzung a) 10, 5 oder 2,5 mg Ramipril, berechnet auf eine freie Ramipril-Säure, umfasst, und die Schicht zur Langzeitfreisetzung b) 1,5 mg Indapamid, berechnet auf freies Indapamid-Amid, umfasst; vorzugsweise umfasst die Schicht zur sofortigen Freisetzung a) 10 oder 5 mg Ramipril und die Schicht zur Langzeitfreisetzung b) 1,5 mg Indapamid.

5. Pharmazeutische Zusammensetzung nach einem der Ansprüche 1 bis 4, wobei die Schicht zur Langzeitfreisetzung b) 25 bis 45 Gew.-% des Mittels zur Langzeitfreisetzung, vorzugsweise 30 bis 42 Gew.-% und stärker bevorzugt 32 bis 37 Gew.-%, wie beispielsweise 32 Gew.-% des Mittels zur Langzeitfreisetzung, umfasst, bezogen auf das Gesamtgewicht der Schicht zur Langzeitfreisetzung b).

6. Pharmazeutische Zusammensetzung nach einem der Ansprüche 1 bis 5, wobei das HPMC mindestens eines ausgewählt aus der Gruppe bestehend aus Hydroxypropylmethylcellulose (HMPC) der Grade K4M oder E4M ist, und das HEC Hydroxyethylcellulose des Grades HHX ist oder eine Kombination davon.

7. Pharmazeutische Zusammensetzung nach einem der Ansprüche 1 bis 6, wobei die Schicht zur sofortigen Freisetzung a) als den mindestens einen Hilfsstoff mindestens einen der folgenden umfasst: einen Füllstoff, ein Sprengmittel, ein Bindemittel und ein Schmiermittel und eine beliebige Kombination davon, vorzugsweise umfasst die Schicht zur sofortigen Freisetzung b) 80-90 Gew.-% eines Füllstoffs, 0,5-3,0 Gew.-% eines Schmiermittels und 0,75 bis 5 Gew.-% eines Bindemittels, bezogen auf das Gesamtgewicht der Schicht zur sofortigen Freisetzung a).

8. Pharmazeutische Zusammensetzung nach einem der Ansprüche 1 bis 7, wobei die Schicht zur Langzeitfreisetzung b) als den mindestens einen Hilfsstoff mindestens einen der folgenden umfasst: einen Füllstoff, ein Mittel zur Langzeitfreisetzung, ein Bindemittel, ein Schmiermittel und ein Gleitmittel und eine beliebige Kombination davon, vorzugsweise umfasst die Schicht zur Langzeitfreisetzung b) 25 bis 45 Gew.-% des Mittels zur Langzeitfreisetzung, 50 bis 70 Gew.-% eines Füllstoffs, 4,0 bis 5,0 Gew.-% eines Bindemittels, 0,2 bis 0,6 Gew.-% eines Schmiermittels und 0,1 bis 0,5 Gew.-% eines Gleitmittels, bezogen auf das Gesamtgewicht der Schicht zur Langzeitfreisetzung b).

9. Pharmazeutische Zusammensetzung nach einem der Ansprüche 1 bis 8, wobei die pharmazeutische Zusammensetzung eine in-vitro-Auflösungsrate von Indapamid in einem Bereich von 10 bis 30 Gew.-%, vorzugsweise 12 bis 19 Gew.-% Indapamid innerhalb von 2 h, 40 bis 65 Gew.-% innerhalb von 8 h, 45 bis 65% innerhalb von 10 h und über 85 Gew.-% innerhalb von 21 h, bezogen auf den Gesamtgehalt an Indapamid, bestimmt nach der Europäischen Pharmakopöe, Ausgabe 8, unter Verwendung von Apparatepaddeln "2", in 900 ml Phosphatpuffer pH 6,8 bei 37,0°C±0,5°C und 50 rpm, und eine Auflösungsrate von Ramipril von 85 Gew.-%, vorzugsweise 90 Gew.-%, am meisten bevorzugt 97 Gew.-% von Ramipril innerhalb von 15 Minuten, bezogen auf den Gesamtgehalt an Ramipril, bestimmt nach der Europäischen Pharmakopöe, Ausgabe 8, unter Verwendung von Apparatekörben "1" in 500 ml 0,1 M HCl (pH 1,2) bei 37,0°C±0,5°C und 100 rpm, aufweist.

10. Pharmazeutische Zusammensetzung nach einem der Ansprüche 1 bis 9, umfassend in der Schicht zur Langzeitfreisetzung b) 1,5 mg Indapamid, 124,5 mg Lactose-Monohydrat, 8,6 mg Povidon K-30, 64 mg Hypromellose K4M, 0,4 mg kolloidales wasserfreies Siliciumdioxid, 1 mg Magnesiumstearat und in der Schicht zur sofortigen Freisetzung a) 10 mg Ramipril, 176,15 mg Lactosemonohydrat, 10,2 mg Croscarmellose-Natrium, 3,57 mg Polyvinylalkohol und 4,08 mg Stearylfumarat-Natrium;

11. Pharmazeutische Zusammensetzung nach einem der Ansprüche 1 bis 9, umfassend in der Schicht zur Langzeitfreisetzung b) 1,5 mg Indapamid, 124,5 mg Lactose-Monohydrat, 8,6 mg Povidon K-30, 64 mg Hypromellose K4M, 0,4 mg kolloidales wasserfreies Siliciumdioxid, 1 mg Magnesiumstearat und in der Schicht zur sofortigen Freisetzung a) 5 mg Ramipril, 88,08 mg Lactosemonohydrat, 5,1 mg Croscarmellose-Natrium, 1,78 mg Polyvinylalkohol und 2,04 mg Stearylfumarat-Natrium.

## Revendications

1. Composition pharmaceutique pour administration orale sous forme de comprimé, comprenant dans une forme galénique unique :
a) une couche à libération immédiate, comprenant du ramipril et de l'alcool polyvinylique dans un rapport % en poids compris dans une plage de 1,1 à 4,0 et un ou plusieurs excipients pharmaceutiquement acceptables ; et
b) une couche à libération prolongée, comprenant de l'indapamide, un agent à libération prolongée étant l'hydroxypropylméthylcellulose ou l'hydroxyéthylcellulose ayant une viscosité comprise dans la plage de 2 500 à 5 500 mPas, mesurée selon la méthode 2.2.8 de la Pharmacopée européenne 7.0 dans une solution aqueuse à 2 %, et un ou plusieurs excipients pharmaceutiquement acceptables.

2. Composition pharmaceutique selon la revendication 1, dans laquelle dans la couche à libération immédiate a), le rapport en poids du ramipril à l'alcool polyvinylique est compris dans la plage de 1,15 à 3,9, plus préférablement de 1,7 à 2,85, par exemple 2,8.

3. Composition pharmaceutique selon la revendication 1 ou 2, dans laquelle l'hydroxypropylméthylcellulose ou l'hydroxyéthylcellulose a une viscosité comprise dans la plage de 2 700 à 5 500 mPas, plus préférablement de 2 500 à 5 040 mPas, par exemple 4 000 mPas.

4. Composition pharmaceutique selon l'une quelconque des revendications 1 à 3, dans laquelle la couche à libération immédiate a) comprend 10, 5 ou 2,5 mg de ramipril calculé sur un acide libre de ramipril, et la couche à libération prolongée b) comprend 1,5 mg d'indapamide, calculé sur l'amide libre d'indapamide ; de préférence, la couche à libération immédiate a) comprend 10 ou 5 mg de ramipril, et la couche à libération prolongée b) comprend 1,5 mg d'indapamide.

5. Composition pharmaceutique selon l'une quelconque des revendications 1 à 4, dans laquelle la couche à libération prolongée b) comprend 25 à 45 % en poids de l'agent à libération prolongée, de préférence 30 à 42 % en poids et plus préférablement 32 à 37 % en poids, par exemple 32 % en poids de l'agent à libération prolongée, sur la base du poids total de la couche à libération prolongée b).

6. Composition pharmaceutique selon l'une quelconque des revendications 1 à 5, dans laquelle l'HPMC est au moins une choisie dans le groupe constitué d'hydroxypropylméthylcellulose (HMPC) de qualités K4M ou E4M et l'HEC est l'hydroxyéthylcellulose de qualité HHX ou toute combinaison de celles-ci.

7. Composition pharmaceutique selon l'une quelconque des revendications 1 à 6, dans laquelle la couche à libération immédiate a) comprend, comme au moins un excipient, au moins l'un parmi un agent de charge, un désintégrant, un liant et un lubrifiant et toute combinaison de ceux-ci, de préférence, la couche à libération immédiate b) comprend 80 à 90 % en poids d'un agent de charge, 0,5 à 3,0 % en poids d'un lubrifiant et 0,75 à 5 % en poids d'un liant, sur la base du poids total de la couche à libération immédiate a).

8. Composition pharmaceutique selon l'une quelconque des revendications 1 à 7, dans laquelle la couche à libération prolongée b) comprend, en tant que l'au moins un excipient, au moins l'un parmi un agent de charge, un agent à libération prolongée, un liant, un lubrifiant, un agent de glissement et tout combinaison de ceux-ci, de préférence, la couche à libération prolongée b) comprend 25 à 45 % en poids de l'agent à libération prolongée, 50 à 70 % en poids d'un agent de charge, 4,0 à 5,0 % en poids d'un liant, 0,2 à 0,6 % en poids d'un lubrifiant et 0,1 à 0,5 % en poids d'un agent de glissement, sur la base du poids total de la couche à libération prolongée b).

9. Composition pharmaceutique selon l'une quelconque des revendications 1 à 8, dans laquelle la composition pharmaceutique a un taux de dissolution in vitro de l'indapamide qui se situe dans la plage de 10 à 30 % en poids, de préférence de 12 à 19 % en poids d'indapamide en 2 h, 40 à 65 % en poids en 8 h, 45 à 65 % en 10 h et plus de 85 % en poids en 21 h, sur la base de la teneur totale en indapamide telle que déterminée à l'aide de l'édition 8 de la Pharmacopée européenne Palettes d'appareil « 2 » dans un volume de 900 ml de tampon phosphate de pH 6,8 à 37,0 °C ± 0,5 °C et 50 tr/min, et un taux de dissolution du ramipril de 85 % en poids, de préférence de 90 % en poids, plus préférablement de 97 % en poids de ramipril en 15 minutes, sur la base de la teneur totale en ramipril telle que déterminée à l'aide de l'édition 8 de la Pharmacopée européenne Paniers d'appareils « 1 » dans un volume de 500 ml de HCl 0,1 M (pH 1,2) à 37,0 °C ± 0,5 °C et 100 tr/min.

10. Composition pharmaceutique selon l'une quelconque des revendications 1 à 9, comprenant dans la couche à libération prolongée b) 1,5 mg d'indapamide, 124,5 mg de lactose monohydraté, 8,6 mg de povidone K-30, 64 mg d'hypromellose K4M, 0,4 mg de silice, colloïdal anhydre, 1 mg de stéarate de magnésium et dans la couche à libération immédiate a) 10 mg de ramipril, 176,15 mg de lactose monohydraté, 10,2 mg de croscarmellose sodique, 3,57 mg d'alcool polyvinylique et 4,08 mg de fumarate de stéaryle sodique ;

11. Composition pharmaceutique selon l'une quelconque des revendications 1 à 9, comprenant dans la couche à libération prolongée b) 1,5 mg d'indapamide, 124,5 mg de lactose monohydraté, 8,6 mg de povidone K-30, 64 mg d'hypromellose K4M, 0,4 mg de silice, colloïdal anhydre, 1 mg de stéarate de magnésium et dans la couche à libération immédiate a) 5 mg de ramipril, 88,08 mg de lactose monohydraté, 5,1 mg de croscarmellose sodique, 1,78 mg d'alcool polyvinylique et 2,04 mg de fumarate de stéaryle sodique.
